(19) Europäisches Patentamt
European Patent Office
Office européen des brevets

(11) EP 0 906 332 B1

(12) **EUROPÄISCHE PATENTSCHRIFT**

(45) Veröffentlichungstag und Bekanntmachung des Hinweises auf die Patenterteilung:
**28.11.2001 Patentblatt 2001/48**

(21) Anmeldenummer: **97924989.3**

(22) Anmeldetag: **26.05.1997**

(51) Int Cl.[7]: **C07J 41/00**, A61K 31/565, C07J 51/00, C07J 1/00, C07J 17/00, C07J 31/00

(86) Internationale Anmeldenummer:
**PCT/EP97/02718**

(87) Internationale Veröffentlichungsnummer:
**WO 97/45441 (04.12.1997 Gazette 1997/52)**

(54) **7ALPHA-(5-METHYLAMINOPENTYL)-ESTRATRIENE, VERFAHREN ZU DEREN HERSTELLUNG, PHARMAZEUTISCHE PRÄPARATE, DIE DIESE 7ALPHA-(5-METHYLAMINOPENTYL)-ESTRATRIENE ENTHALTEN SOWIE DEREN VERWENDUNG ZUR HERSTELLUNG VON ARZNEIMITTELN**

7ALPHA-(5-METHYLAMINOPENTYL)-ESTRATRIENES, PROCESS FOR THE PREPARATION THEREOF, PHARMACEUTICAL PREPARATIONS WHICH CONTAIN SAID 7ALPHA-(5-METHYLAMINOPENTYL)-ESTRATRIENES AND USE THEREOF FOR PRODUCTION OF DRUGS

7ALPHA-(5-METHYLAMINOPENTYL)-ESTRATRIENES, LEUR PROCEDE DE PRODUCTION, PREPARATIONS PHARMACEUTIQUES CONTENANT CES 7ALPHA-(5-METHYLAMINOPENTYL)-ESTRATRIENES, AINSI QUE LEUR UTILISATION POUR LA PRODUCTION DE MEDICAMENTS

(84) Benannte Vertragsstaaten:
**AT BE CH DE DK ES FI FR GB GR IE IT LI LU MC NL PT SE**
Benannte Erstreckungsstaaten:
**AL LT LV RO SI**

(30) Priorität: **24.05.1996 DE 19622457**

(43) Veröffentlichungstag der Anmeldung:
**07.04.1999 Patentblatt 1999/14**

(73) Patentinhaber: **SCHERING AKTIENGESELLSCHAFT**
**13353 Berlin (DE)**

(72) Erfinder:
- **BITTLER, Dieter**
  **D-13503 Berlin (DE)**
- **BOHLMANN, Rolf**
  **D-14055 Berlin (DE)**
- **HEINRICH, Nikolaus**
  **D-12159 Berlin (DE)**
- **KROLL, Jorg**
  **D-12157 Berlin (DE)**
- **SAUER, Gerhard**
  **D-13465 Berlin (DE)**
- **NISHINO, Yukishige**
  **D-14089 Berlin (DE)**
- **PARCZYK, Karsten**
  **D-12207 Berlin (DE)**
- **SCHNEIDER, Martin**
  **D-13469 Berlin (DE)**
- **HEGELE-HARTUNG, Christa**
  **D-10179 Berlin (DE)**
- **LICHTNER, Rosemarie**
  **D-10823 Berlin (DE)**

(56) Entgegenhaltungen:
**EP-A- 0 138 504** **DE-A- 4 218 743**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 906 332 B1

Printed by Jouve, 75001 PARIS (FR)

## Beschreibung

Die vorliegende Erfindung betrifft 7α-(5-Methylaminopentyl)-estratriene der allgemeinen Formel **I**

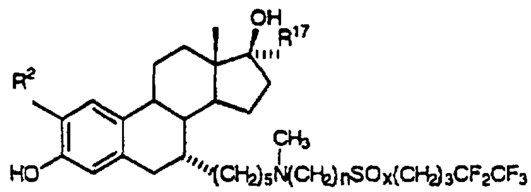

worin

$R^2$ für ein Wasserstoff- oder Fluoratom,
$R^{17}$ für ein Wasserstoffatom, eine Methyl- oder Ethinylgruppe,
n für 2, 3 oder 4 und
x für 0, 1 oder 2

stehen.

Insbesondere betrifft die vorliegende Erfindung die Estratriene

7α-{5-[N-Methyl-N-3-(4,4,5,5,5-pentafluorpentylthio)-propylamino]-pentyl}-estra-1,3,5(10)-trien-3,17β-diol
17α-Methyl-7α-{5-[N-methyl-N-3-(4,4,5,5,5-pentafluorpentylthio)-propylamino]-pentyl}-estra-1,3,5(10)-trien-3,17β-diol
17α-Ethinyl-7α-{5-[N-methyl-N-3-(4,4,5,5,5-pentafluorpentylthio)-propylamino]-pentyl}-estra-1,3,5(10)-trien-3,17β-diol
7α-{5-[N-Methyl-N-2-(4,4,5,5,5-pentafluorpentansulfonyl)-ethylamino]-pentyl}-estra-1,3,5(10)-trien-3,17β-diol
7α-{5-[N-Methyl-N-3-(4,4,5,5,5-pentafluorpentansulfonyl)-propylamino]-pentyl}-estra-1,3,5(10)-trien-3,17β-diol
2-Fluor-7α-{5-[N-Methyl-N-3-(4,4,5,5,5-pentafluorpentylthio)-propylamino]-pentyl}-estra-1,3,5(10)-trien-3,17β-diol
7α-{5-[N-Methyl-N-3-(4,4,5,5,5-pentafluorpentansulfinyl)-propylamino]-pentyl}-estra-1,3,5(10)-trien-3,17β-diol
2-Fluor-17α-methyl-7α-{5-[N-Methyl-N-3-(4,4,5,5,5-pentafluorpentylthio)-propylamino]-pentyl}-estra-1,3,5(10)-trien-3,17β-diol
2-Fluor-7α-{5-[N-Methyl-3-(4,4,5,5,5-pentafluorpentansulfinyl)-propylamino]-pentyl}-estra-1,3,5(10)-trien-3,17β-diol
2-Fluor-17α-methyl-7α-{5-[N-Methyl-N-3-(4,4,5,5,5-pentafluorpentansulfonyl)-propylamino]-pentyl}-estra-1,3,5(10)-trien-3,17β-diol
7α-{5-[N-Methyl-N-4-(4,4,5,5,5-pentafluorpentylthio)-butylamino]-pentyl}-estra-1,3,5(10)-trien-3,17β-diol
7α-{5-[N-Methyl-N-4-(4,4,5,5,5-pentafluorpentansulfinyl)-butylamino]-pentyl}-estra-1,3,5(10)-trien-3,17β-diol
17α-Methyl-7α-{5-[N-methyl-N-3-(4,4,5,5,5-pentafluorpentansulfinyl)-propylamino]-pentyl}-estra-1,3,5(10)-trien-3,17β-diol
17α-Methyl-7α-{5-[N-methyl-N-3-(4,4,5,5,5-pentafluorpentansulfonyl)-propylamino]-pentyl}-estra-1,3,5(10)-trien-3,17β-diol
17α-Ethinyl-7α-{5-[N-methyl-N-3-(4,4,5,5,5-pentafluorpentansulfonyl)-propylamino]-pentyl}-estra-1,3,5(10)-trien-3,17β-diol

Die Verbindungen der allgemeinen Formel I stellen Verbindungen mit sehr starker antiestrogener Wirksamkeit dar.

Verbindungen mit antiestrogenen Eigenschaften, d.h. Stoffe mit Hemmwirkungen gegenüber Estrogenen, sind bereits zahlreich beschrieben worden.
Als den vorliegenden Verbindungen der allgemeinen Formel I strukturell am nächsten kommende Verbindungen sind die aus der EP-A 0 138 504 hervorgehenden Steroid-Derivate anzusehen, und von diesen insbesondere das 7α-[9-(4,4,5,5,5-Pentafluorpentansulfinyl)-nonyl]-estra-1,3,5(10)-trien-3,17β-diol (Seite 58, vorletzte Verbindung). Diese Verbindung befindet sich gegenwärtig in klinischer Entwicklung für hormonabhängige Tumoren (Brustkrebs) und stellt die derzeit beste bekannte Verbindung, d.h. diejeniege mit der stärksten antiestrogenen Wirksamkeit, dieser Steroid-Derivate dar.

Bei den erfindungsgemäßen Verbindungen handelt es sich um reine Antiestrogene mit stärkerer, zum Teil

mehrfach besserer, antiestrogener Wirkung als 7α-[9-(4,4,5,5,5-Pentafluorpentansulfinyl)-nonyl]-estra-1,3,5(10)-trien-3,17β-diol. Dies gilt für mehrere Kriterien *in vitro* und *in vivo,* besonders aber für die orale Wirkung in vivo an der Ratte.

Pharmazeutische Zusammensetzungen, die Sexualsteroid-Inhibitoren enthalten, welche ein steroidales Grundgerüst, das u.a. eine 7α-Seitenkette bei gleichzeitiger Anwesenheit eines weiteren Substituenten aufweist, sind Gegenstand der EP-A 0 367 576.

Weitere steroidale Antiestrogene sind in den EP-AS 0 384 842 und 0 629 635 beschrieben, die einen 11β-Phenylrest tragen.

Eine Vielzahl verschiedenartigster Verbindungen - u. a. solche steroidalen Ursprungs als auch solche mit 2-Phenylindol-Grundgerüst - die als Antiestrogen wirken und/oder die Estrogenbiosynthese unterdrücken, werden in der WO 93/10741 offenbart.

Die Verbindungen der allgemeinen Formel I gemäß vorliegender Anmeldung zeichnen sich im Vergleich zu den bereits bekannten Steroid-Derivaten gemäß der EP-A 0 138 504 durch neuartige Seitenketten am Kohlenstoffatom 7 des Steroid-Gerüsts aus. Diese Strukturmodifikation führt zu besonders hoch antiestrogen wirksamen Verbindungen.

Die antiestrogene Wirkung der erfindungsgemäßen Verbindungen wurde in Transaktivierungsassays bestimmt [Demirpence E., Duchesne M.-J., Badia E., Gagne D. und Pons M.: MVLN Cells: A Bioluminescent MCF-7-Derived Cell Line to study the Modulation of Estrogenic Activity; J. Steroid. Molec. Biol. Vol. 46, No. 3, 355 - 364(1993) sowie Berry M., Metzger D. Chambon P.: Role of the two activating domains of the estrogen receptor in the cell-type and promoter-context dependent agonistic activity of the anti-estrogen 4-hydroxytamoxifen; The EMBO Journal Vol. 9, 2811 - 2818 (1990)].

Die antiproliferative Aktivität der neuen Verbindungen in Mammakarzinom-Zellinien ist höher als die des 7α-[9-(4,4,5,5,5-Pentafluorpentansulfinyl)-nonyl]-estra-1,3,5(10)-trien-3,17β-diols.

Die $IC_{50}$-Werte für die neuen Verbindungen liegen im nanomolaren Bereich. In der Hela- sowie der MVLN-Zellinie ergeben sich für die Verbindungen der Beispiele 1 bis 6 sowie für die Referenzverbindung 7α-[9-(4,4,5,5,5-Pentafluorpentansul finyl)-nonyl]-estra-1,3,5(10)-trien-3,17β-diol folgende $IC_{50}$-Werte (Testdurchführung gemäß der oben angegebenen Literaturstellen). Die Hela-Zellen sind mit humanem Estrogenrezeptor-Expressionsvektor (HEGO) und Vit-TK-CAT Reportergen und die MVLN-Zellen mit dem Reportergen Vit-TK-LUC transfiziert:

| Verbindung | $IC_{50}$ [nM] | |
|---|---|---|
| | Hela-Zellen | MVLN.Zellen |
| Beispiel 1 | 0,3 | 1,0 |
| Beispiel 2 | 0,2 | 0,6 |
| Beispiel 3 | 1,0 | 0,6 |
| Beispiel 4 | 0,16 | 1,3 |
| Beispiel 5 | 0,3 | 0,66 |
| Beispiel 6 | 0,1 | 1,0 |
| Referenz: | | |
| 7α-[9-(4,4,5,5,5-Pentafluorpentansulfinyl)nonyl]-estra-1,3,5(10)-trien-3,17β-diol | 0,5 | 6,0 |

Die Verbindungen wirken hemmend auf das Wachstum von hormonabhängigen Tumorzellen, insbesondere hemmen sie das Wachstum von estrogen-abhängigen menschlichen Mammatumorzellen (MCF-7).

Ebenso belegen in-vivo Tests eindrucksvoll die Überlegenheit der erfindungsgemäßen Verbindungen gegenüber dem 7α-[9-(4,4,5,5,5-Pentafluorpentansulfinyl)-nonyl]-estra-1,3,5(10)-trien-3,17β-diol. Die nachfolgend beschriebenen Tests wurden durchgeführt:

1. Uteruswachstumstest bei der infantilen Ratte, s.c. und p.o. (Test auf antiestrogene Wirkung)

2. Tumortests Antitumorwirkung auf das hormonabhängige Mammakarzinom)

    2.1. MXT-Mammatumor bei der Maus
    2.2. DMBA*-induziertes Mammakarzinom bei der Ratte
    2.3. NMU⁺-induziertes Mammakarzinom bei der Ratte

* = Dimethylbenzanthrazen
⁺ = N-Nitroso-N-methylharnstoff

**1. Uteruswachstumstest bei der infantilen Ratte (antiestrogene Wirkung)**

Prinzip der Methode

Bei Nagern reagiert der Uterus auf die Applikation von estrogenen mit einer Gewichtszunahme (sowohl Proliferation als auch Wassereinlagerung). Dieses Wachstum ist durch gleichzeitige Gabe antiestrogen-wirkender Verbindungen dosisabhängig zu hemmen.

**Versuchsdurchführung**

Tiere:

Infantile weibliche Ratten im Gewicht von 35-45 g bei Versuchsbeginn, pro Dosis 5-6 Tiere.

Formulierung und Applikation der Substanzen:

Sowohl für die s.c. als auch für die p.o. Applikation werden die Substanzen in 1 Teil Ethanol (E) gelöst und mit 9 Teilen Erdnußöl (EÖ) aufgefüllt.

Versuchsansatz

Die gerade von den Müttern abgesetzten jungen Ratten werden zur Eingewöhnung einen Tag vor Behandlungsbeginn geliefert und sofort mit Futter - auch in dem Tierkäfig - versorgt.
Die Behandlung erfolgt dann täglich einmal über 3 Tage in Kombination mit 0,5 µg Estradiolbenzoat (EB). EB wird immer subcutan (s.c.) appliziert, während die Testsubstanz entweder s.c. oder p.o. (peroral) verabreicht wird. 24 Stunden nach der letzten Applikation werden die Tiere gewogen, getötet und die Uteri entnommen. Von den präparierten Uteri werden die Feuchtgewichte (ohne Inhalt) ermittelt.

Kontrollen

negative Kontrolle: Vehikel (E/EÖ), 0,2 ml/Tier/Tag
positive Kontrolle: 0,5 µg EB/0,1 ml/Tier/Tag

Auswertung

Von den reltiven Organgewichten (mg/100 g Körpergewicht) werden für jede Gruppe die Mittelwerte mit Standardabweichung (X+SD), sowie die Signifikanz der Unterschiede zur Kontrollgruppe (EB) im Dunnett-Test ($p<0.05$) ermittelt. Die Berechnung der Hemmung (in %) gegenüber der EB-Kontrolle erfolgt mit einem Programm. Die relativen Wirksamkeiten der Prüfsubstanzen werden durch eine Kovarianz- und Regressionsanalyse ermittelt.

**2. Tumortests**

**2.1. Tumorhemmende Wirkung am hormonabhängigen MXT(+)-Mammakarcinom der Maus**

Prinzip der Methode

Das MXT(+)-Mammakarcinom ist ein estrogen- und progesteronrezeptor-positiver Tumor, der ursprünglich durch Induktion mit Urethan erzeugt wurde und seither seriell weitertransplantiert wird. Das Wachstum ist durch Ovariektomie und Antiestrogene hemmbar. Daher eignet sich dieses Modell sehr gut zur Untersuchung der tumorhemmenden Wirkung von Endokrintherapeutika.

**Versuchsdurchführung**

Tiermaterial

Adulte, weibliche BDF1-Mäuse (7-10 Wochen alt; Gewicht 18-21 g). Standardhaltungsbedingungen.

Formulierung und Applikation der Testsubstanzen

Die Testsubstanzen werden in Ethanol + Erdnußöl (1+9) gelöst und die einmalige Dosis in einem Volumen von 0,1 ml s.c. appliziert. Bei oraler Anwendung wird die Testsubstanz in Ethanol + Erdnußöl (1+9) gelöst und die Tagesdosis in einem Volumen von 0,1 ml verabreicht. Die Therapie beginnt entweder sofort nach Tumorimplantation oder 2-3 Wochen danach (Therapie etablierter Tumoren). Die maximale Behandlungszeit beträgt 12 Wochen.

Tumorstammhaltung

Dazu werden Tumorfragmente von ca. 2 mm Kantenlänge inguinal s.c. beidseitig intakten Mäusen mit einer Lumbalpunktationskanüle implantiert. Je nach Wachstum des Tumors wird alle 5-6 Wochen eine neue Stammerhaltung begonnen.

Versuchsansatz

Die Tumoren werden von mindestens 4 Tumoren der Stammerhaltung unter aseptischen Bedingungen entnommen und gepoolt, in Stücke von ca. 2 mm Kantenlänge geschnitten und den Tieren wie oben beschrieben implantiert. Die Mäuse werden zu 9-10 Tieren pro Gruppe randomisiert. Eine Gruppe wird zur Kontrolle der Hormonabhängigkeit ovariektomiert. Das Tumorwachstum wird durch Ermittlung der Tumorfläche mittels einer Schiebelehre bestimmt. Die Tumorfläche errechnet sich aus dem Produkt des längsten und des darauf senkrecht stehenden Durchmessers des Tumors. Zu Versuchsende werden die Tiere getötet, die Tumoren sowie ggf. Uteri und Ovarien herauspräpariert und gewogen.

Auswertung

Mittelwerte +/- SD von Tumorflächen werden ermittelt. Der Wachstumsverlauf der Tumoren wird graphisch dargestellt.
Die statistische Auswertung erfolgt mit dem Dunnett-Test.

**2.2. Beeinflussung des Tumorwachstums im DMBA*-Modell bei der Ratte (DMBA-Tumormodell)**

Biologische Grundlage

Das Wachstum des DMBA-induzierten Mammatumors der Ratte ist weitgehend von Estrogenen und Prolaktin abhängig. Wirksame Antiestrogene, Antigestagene und Aromatasehemmer führen zur Hemmung des Tumorwachstums. Substanzen, die antigonadotrope und androgene Eigenschaften besitzen, entfalten ebenfalls tumorhemmende Wirkung.

Tiermaterial

45 - 47 Tage alte weibliche Ratten (Sprague-Dawley, Züchter ZIH oder Möllegard), pro Gruppe 8 - 10 Tiere.

Versuchsansatz

Die Tiere erhalten einmalig 10 mg DMBA oral. Anschließend werden die Tiere einmal wöchentlich palpatorisch auf Tumorentwicklung untersucht. 6 bis 10 Wochen nach der DMBA-Behandlung entwickeln sich etwa 1 bis 10 Tumoren pro Tier. Die Tumorgröße wird einmal wöchentlich mit Hilfe einer Schiebelehre bestimmt. Hat mindestens ein Tumor eine definierte Größe (150 $mm^2$ Tumorfläche) erreicht, erfolgt die Ovariektomie der Tiere bzw. beginnt die Behandlung der Tiere mit der Testsubstanz. Die Behandlung erfolgt zumeist täglich über ca. 28 Tage (Details Abwicklung siehe Versuchsplan). Die Tumorgröße wird weiterhin 1 x /Woche ermittelt.

Auswertung

Die Gesamttumorgröße pro Tier wird vor Beginn der Behandlung ermittelt (Vorwerte). Für jede Gruppe wird dann der Mittelwert der prozentualen Veränderungen der Tumorgröße, bezogen auf die initialen Werte, errechnet. Außerdem wird der Prozentsatz an Tieren pro Gruppe ermittelt, deren Tumoren jeweils (1) total zurückgegangen (totale Regression), (2) teilweise zurückgegangen (partiale Regresssion), (3) unverändert (keine Veränderung) bzw. (4) weiter

*9,10-Dimethyl-1,2-benzanthracen

vergrößert (Vergrößerung) sind.

Die ermittelten Werte werden im Dunnett-Test auf Signifikanz getestet und grafisch dargestellt.

**2.3 Beeinflussung des Tumorwachstums im NMU⁺-Modell bei der Ratte (NMU-Tumormodell)**

Biologische Grundlage

Das Wachstum des NMU-induzierten Mammatumors der Ratte ist weitgehend von Estrogenen und im Gegensatz zum DMBA-Tumormodell weniger von Prolaktin abhängig. Wirksame Antioestrogene, Aromatasehemmer und Antigestagene führen zur Hemmung des Tumorwachstums. Substanzen mit antigonadotropen Eigenschaften entfalten ebenfalls tumorhemmende Wirkung.

Tiermaterial

Weibliche Ratten (Sprague-Dawley)
55 d alt (± 3 d)
pro Gruppe 8 - 10 Tiere

Applikationsart

NMU = i.v., Testsubstanz : s.c. / p.o.

| Vehikelvolumen | |
|---|---|
| p.o. = 0,1 oder 0,2 ml/100 g/d | in Ethanol + Erdnußöl (1+9) |
| s.c. = 0,1 ml/100 g/d | in Ethanol + Erdnußöl (1+9) |
| NMU = 50 mg/kg/d | 1,5 ml/200 g (0,9% NaCl/0.085% Myrj-53) |

Versuchsansatz

Die Tiere erhalten einmalig 50 mg/kg NMU i.v. Anschließend werden die Tiere einmal wöchentlich palpatorisch auf Tumorentwicklung untersucht. Ca. 6-8 Wochen nach NMU-Behandlung entwickeln sich 1 oder mehrere Tumore pro Tier. Bei einer Mindestgröße von 150 $mm^2$ /Tumor/Tier beginnt die tägliche Behandlung (7xWoche) mit der Testsubstanz und die Ermittlung des Körpergewichts + der Tumorgröße (mit Hilfe einer Schiebelehre) (1x/Woche).

Die ermittelten Werte werden im Dunnett-Test auf Signifikanz getestet und grafisch dargestellt.

**Versuchsergebnisse**

**1. Antiuterotrope Wirkung bei der infantilen Ratte**

Bei subcutaner bzw. oraler Applikation ist 7α-{5-[N-Methyl-N-3-(4,4,5,5,5-pentafluorpentylthio)-propylamino]-pentyl}-estra-1,3,5(10)-trien-3,17β-diol jeweils 5.4mal bzw. 4.7mal stärker antiestrogen wirksam als 7α-[9-(4,4,5,5,5-Pentafluorpentansulfinyl)-nonyl]-estra-1,3,5(10)-trien-3,17β-diol (Abb. 1 und 2).
Diese Unterscheide der Wirksamkeit sind hoch signifikant.

**2. Antitumorwirkung auf das hormonabhängige Mammakarzinom**

**2.1. MXT-Mammatumor bei der Maus**

Nach 14tägiger Behandlung mit einer oralen Dosis von 10 mg/kg/Tag hemmt 7α-{5-[N-Methyl-N-3-(4,4,5,5,5-pentafluorpentylthio)-propylamino]-pentyl}-estra-1,3,5(10)-trien-3,17β-diol das Tumorwachstum ähnlich stark wie eine Ovariektomie (Abb. 3). 7α-{5-[N-Methyl-N-3-(4,4,5,5,5-pentafluorpentylthio)-propylamino]-pentyl}-estra-1,3,5(10) trien-3,17β-diol ist dabei stärker wirksam als 7α-[9-(4,4,5,5,5-Pentafluorpentansulfinyl)-nonyl]-estra-1,3,5(10)-trien-3,17β-diol.

⁺N-Methyl-N-nitrosohamstoff

### 2.2. DMBA-induziertes Mammakarzinom bei der Ratte

Bei einer oralen Dosis von 3 mg/kg/Tag hemmt 7α-{5-[N-Methyl-N-3-(4,4,5,5,5-pentafluorpentylthio)-propylamino]-pentyl}-estra-1,3,5(10)-trien-3,17β-diol das Tumorwachstum vollständig (kein Wachstum), während 7α-[9-(4,4,5,5,5-Pentafluorpentansulfinyl)-nonyl]-estra-1,3,5(10)-trien-3,17β-diol bei der gleichen Dosis nur geringfügig wirksam ist (Abb. 4). 7α-{5-[N-Methyl-N-3-(4,4,5,5,5-pentafluorpentylthio)-propylamino]-pentyl}-estra-1,3,5(10)-trien-3,17β-diol ist auch stärker wirksam als Tamoxifen (TAM). Ovariektomie führt dabei zu einer totalen Remission der Tumoren.

### 2.3. NMU-induziertes Mammakarzinom bei der Ratte

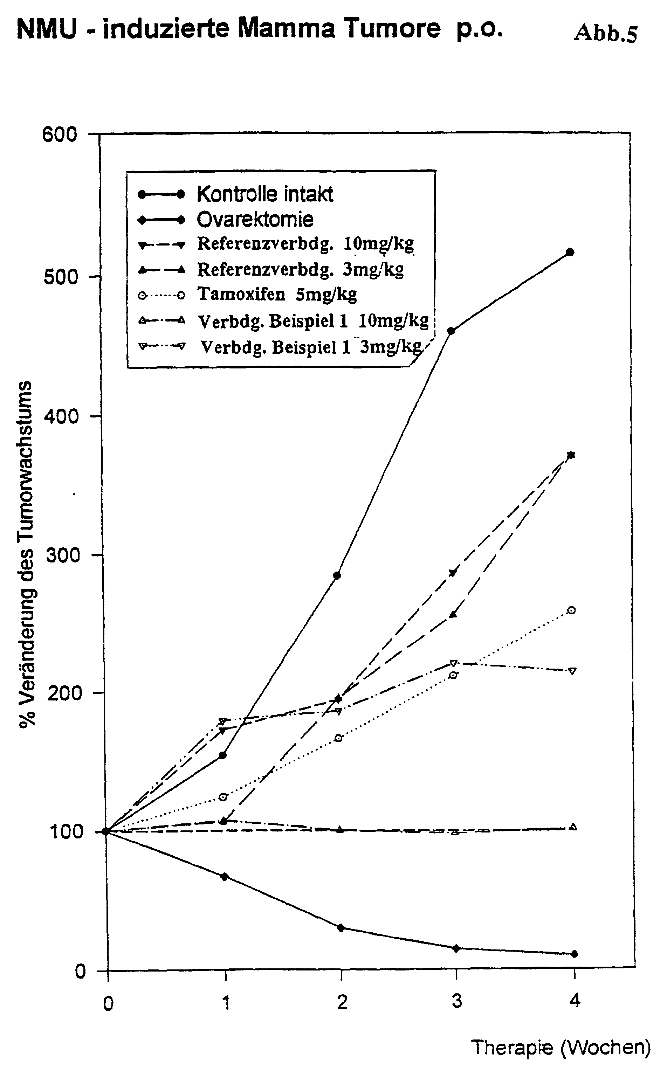

7α-{5-[N-Methyl-N-3-(4,4,5,5,5-pentafluorpentylthio)-propylamino]-pentyl}-estra-1,3,5(10)-trien-3,17β-diol, oral verabreicht, hemmt das Tumorwachstum dosisabhängig (Abb. 5). Bei einer Dosis von 10 mg/kg/Tag führt 7α-{5-[N-Methyl-N-3-(4,4,5,5,5-pentafluorpentylthio)-propylamino]-pentyl}-estra-1,3,5(10)-trien-3,17β-diol zu einer vollständigen Hemmung des Tumorwachstums. 7α-{5-[N-Methyl-N-3-(4,4,5,5,5-pentafluorpentylthio)-propylamino]-pentyl}-estra-1,3,5(10)-trien-3,17β-diol ist deutlich stärker wirksam als 7α-[9-(4,4,5,5,5-Pentafluorpentansulfinyl)-nonyl]-estra-1,3,5(10)-trien-3,17β-diol.

Die erfindungsgemäßen Verbindungen eignen sich somit zur Therapie von estrogen-abhängigen Erkrankungen, zum Beispiel Mammakarcinom, Endometriumkarcinom, Prostatahyperplasie, anovulatorische Infertilität und Melanom.

Die Erfindung betrifft auch pharmazeutische Präparate, die mindestens eine Verbindung der allgemeinen Formel I enthalten und die Verwendung dieser Verbindung zur Herstellung von Arzneimitteln, insbesondere zur Behandlung von estrogenabhängigen Krankheiten und Tumoren.

Die erfindungsgemäßen Verbindungen sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemäßen Verbindungen, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche infrage, die in folgenden Literaturstellen als Hilfsstoffe für Pharmazie, Kosmetik und angrenzende Gebiete empfohlen beziehungsweise angegeben sind: Ullmans Encyklopädie der technischen Chemie, Band 4 (1953), Seite 1 bis 39; Journal of Pharmaceutical Sciences, Band 52 (1963), Seite 918 u. ff.; H.v. Czetsch-Lindenwald, Hilfsstoffe für Pharmazie und angrenzende Gebiete; Pharm. Ind. Heft 2, 1961, Seite 72 u. ff.; Dr. H.P. Fiedler, Lexikon der Hilfsstoffe für Pharmazie, Kosmetik und angrenzende Gebiete Cantor KG. Aulendorf in Württemberg 1971.

Die Verbindungen können bevorzugt oral oder aber auch parenteral, beispielsweise intraperitoneal, intramuskulär, subkutan oder perkutan, verabreicht werden. Die Verbindungen können auch in das Gewebe implantiert werden. Die zu verabreichende Menge der Verbindungen schwankt innerhalb eines weiten Bereichs und kann jede wirksame Menge abdecken. In Abhängigkeit des zu behandelnden Zustandes und der Art der Verabreichung kann die Menge der verabreichten Verbindung 0,1-25 mg/kg Körpergewicht, vorzugsweise 0,5-5 mg/kg Körpergewicht, je Tag betragen. Beim Menschen entspricht dies einer täglichen Dosis von 5 bis 1250 mg.

Zur oralen Verabreichung kommen Kapseln, Pillen, Tabletten, Dragèes usw. infrage. die Dosierungseinheiten können neben dem Wirkstoff einen pharmazeutisch verträglichen Träger, wie zum Beispiel Stärke, Zucker, Sorbit, Gelantine, Gleitmittel, Kieselsäure, Talkum usw., enthalten. Die einzelnen Dosierungseinheiten für die orale Applikation können beispielsweise 5 bis 500 mg des Wirkstoffs enthalten.

Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiermittels verwendet. Beispiele für verwendete Öle sind Olivenöl, Erdnußöl, Baumwollsamenöl, Sojabohnenöl, Rizinusöl und Sesamöl.

Die Verbindungen lassen sich auch in Form einer Depotinjektion oder eines Implantatpräparats anwenden, die so formuliert sein können, daß eine verzögerte Wirkstoff-Freigabe ermöglicht wird.

Implantate können als inerte Materialien zum Beispiel biologisch abbaubare Polymere enthalten oder synthetische Silikone wie zum Beispiel Silikonkautschuk. Die Wirkstoffe können außerdem zur perkutanen Applikation zum Beispiel in ein Pflaster eingearbeitet werden.

Die erfindungsgemäßen Verbindungen können wie nachstehend beschrieben hergestellt werden. Die nachfolgenden Beispiele dienen der näheren Erläuterung der Erfindung. Durch analoge Vorgehensweise unter Verwendung analoger Reagenzien zu den in den Beispielen enthaltenen Angaben lassen sich alle Verbindungen der allgemeinen Formel I erhalten.

**Beispiele**

**Beispiel 1**

**7α-{5-[N-Methyl-N-3-(4,4,5,5,5-pentafluorpentylthio)-propylamino]-pentyl}-estra-1,3,5(10)-trien-3,17β-diol**

**a) 17β-Acetoxy-7α(5-tert. butyl-dimethylsilyloxypentyl)-estr-4-en-3-on**

In 200 ml abs. THF werden 22,9 g Mg-Späne mit 261 g 1-Brom-5-*tert*-butyl-dimethylsilyloxypentan [Tetrahedron Letters 23, 1982, 40, 4147-4150] gelöst in 250 ml abs. THF zum Grignardreagenz umgesetzt. In diese auf -20°C gekühlte Lösung werden unter einem Stickstoffstrom 92,9 g Kupfer-(I)-iodid gegeben und anschließend innerhalb einer Stunde 73,5 g 17β-Acetoxyestra-4,6-dien-3-on [J. Am. Chem. Soc. 80, 1958, 2596-2597] gelöst in 300 ml abs. THF zugetropft. Zur Aufarbeitung werden 61,2 ml Essigsäure zugetropft, die Reaktionsmischung mit Essigester verdünnt, mit gesättigter Ammoniumchloridlösung, Wasser und Natriumhydrogencarbonatlösung gewaschen und getrocknet. Der nach dem Eindampfen erhaltene Rückstand wird an Kieselgel chromatographiert. Man erhält 48 g 17β-Acetoxy-7α-(5-*tert*-butyl-dimethylsilyloxypentyl)-estr-4-en-3-on.

**b) 17β-Acetoxy-7α-(5-hydroxypentyl)-estr-4-en-3-on**

Eine Lösung von 48 g 17β-Acetoxy-7α-(5-*tert*-butyl-dimethylsilyloxypentyl)-estr-4-en-3-on in 350 ml Methanol wird mit 35 ml einer 8 vol.%iger Schwefelsäure 30 Minuten bei Raumtemperatur stehengelassen. Die Lösung wird mit Diethylether verdünnt, mit Wasser neutral gewaschen, getrocknet. Nach dem Eindampfen werden 37,7 g 17β-Acetoxy-7α-(5-hydroxypentyl)-estr-4-en-3-on als Öl erhalten.

**c) 17β-Acetoxy-7α-(5-acetoxypentyl)-estr-4-en-3-on**

Eine Lösung von 37,7 g 17β-Acetoxy-7α-(5-hydroxypentyl)-estr-4-en-3-on in 160 ml Pyridin wird mit 80 ml Acetanhydrid langsam versetzt und 16 h bei 25 °C gerührt. Dann wird mit Essigester verdünnt, die organ. Phase nach dem Waschen mit Natruimhydrogencarbonatlösung, getrocknet und eingedampft. Der Rückstand wird an Kieselgel chromatographiert und es werden 26,6 g 17β-Acetoxy-7α-(5-acetoxypentyl)-estr-4-en-3-on als Öl erhalten. $[\alpha]_D^{22}$ = +20,0° (c = 0,51% in Chloroform)

**d) 17β-Acetoxy-7α-(5-acetoxypentyl)-estra-1,3,5(10)-trien-3-ol**

Zu einer auf 80°C erwärmten Lösung von 26,6 g 17β-Acetoxy-7α-(5-acetoxypentyl)-estr-4-en-3-on in 260 ml Acetonitril wird eine Lösung von 5,18 g Lithiumbromid und 26,73 g Kupfer-II-bromid in 260 ml Acetonitril innerhalb 30 Min unter Rühren zugetropft. Nach beendeter Zugabe wird die Reaktionslösung abgekühlt, mit Diethylether verdünnt, mit Wasser und Natriumhydrogencarbonatlösung gewaschen und getrocknet. Der nach dem Eindampfen erhaltene Rückstand wird an Kieselgel chromatographiert und es werden 21,3 g 17β-Acetoxy-7α-(5-acetoxypentyl)-1,3,5(10)-estratrien-3-ol als Öl erhalten. $[\alpha]^{22}{}_D$ = +28,9° (c = 0,535% in Chloroform)

**e) 17β-Acetoxy-7α-(5-acetoxypentyl)-3-(tetrahydropyran-2-yloxy)-estra-1,3,5(10)-trien**

Eine Lösung von 21,3 g 17β-Acetoxy-7α-(5-acetoxypentyl)-estra-1,3,5(10)-trien-3-ol in 213 ml Tetrahydrofuran wird mit 21,3 ml 3,4-Dihydro-2H-pyran und 1,065 g *p*-Toluolsulfonsäure 8 Stunden bei Raumtemperatur stehengelassen. Die Reaktionslösung wird mit 3 ml Pyridin versetzt, dann mit Diethylether verdünnt, mit Wasser gewaschen und getrocknet. Der nach dem Eindampfen erhaltenen Rückstand wird an Kieselgel chromatographiert und es werden 24,3 g 17β-Acetoxy-7α-(5-acetoxypentyl)-3-(tetrahydropyran-2-yloxy)-estra-1,3,5(10)-trien als Öl erhalten. $[\alpha]_D^{22}$ = +31,5° (c = 0,535% in Chloroform)

**f) 17β-Acetoxy-7α-(5-hydroxypentyl)-3-(tetrahydropyran-2-yloxy)-estra-1,3,5(10)-trien**

Eine Lösung von 10,2 g 17ß-Acetoxy-7α-(5-acetoxypentyl)-3-(tetrahydropyran-2-yloxy)-estra-1,3,5(10)-trien in 205 ml Methanol wird mit 33,7 ml Natronlauge 45 Minuten bei 15 °C gerührt. Dann wird mit Diethylether verdünnt, mit Wasser gewaschen, über Natriumsulfat getrocknet und i.Vak. eingeengt. Man erhält 5.6 g 17β-Acetoxy-7α-(5-hydroxypentyl)-3-(tetrahydropyran-2-yloxy)-estra-1,3,5(10)-trien. $[\alpha]_D^{22}$ = +32,2° (c = 0.505% in Chloroform)

**g) 17β-Acetoxy-3-(tetrahydropyran-2-yloxy)-7α-(5-*p*-toluolsulfonyloxypentyl)-estra-1,3,5(10)-trien**

Eine Lösung von 5,5 g 17β-Acetoxy-7α-(5-hydroxypentyl)-3-(tetrahydropyran-2-yloxy)-estra-1,3,5(10)-trien in 47 ml Pyridin wird mit 5,5 g *p*-Toluolsulfonsäureanhydrid 45 Minuten bei Raumtemperatur stehengelassen. Anschließend wird die Reaktionslösung im Eisbad abgekühlt, mit 4 ml Wasser versetzt und 45 Minuten nachgerührt. Es wird dann mit Essigester verdünnt, mit Wasser gewaschen, getrocknet und eingedampft. Es werden 8,2 g 17β-Acetoxy-3-(tetrahydropyran-2-yloxy)-7α-(5-*p*-toluolsulfonyloxypentyl)-estra-1,3,5,(10)-trien als Öl erhalten.

**h) 17β-Acetoxy-7α-(5-methylaminopentyl)-3-(tetrahydropyran-2-yloxy)-estra-1,3,5(10)-trien**

In eine Lösung von 8,2 g 17β-Acetoxy-3-(tetrahyropyran-2-yloxy)-7α-(5-*p*-toluolsulfonyloxypentyl)-estra-1,3,5(10)-trien in 80 ml Tetrahydrofuran werden in ein Druckrohr 6,3 g Methylamin bei Eiskühlung einkondensiert. Das verschlossene Druckrohr wird dann 6 Stunden auf 60°C erhitzt. Nach dem Abkühlen wird i. Vakuum zur Trockene eingedampft und der Rückstand an Kieselgel chromatographiert. Es werden 5,1 g 17β-Acetoxy-7α-(5-methylaminopentyl)-3-(tetrahydropyran-2-yloxy)-estra-1,3,5(10)-trien als Öl erhalten. $[\alpha]_D^{22}$ = +29,7° (c = 0.535% in Chloroform)

**i) 17β-Acetoxy-7α-{5-[N-methyl-N-3-(4,4,5,5,5-pentafluorpentylthio)-propylamino]-pentyl}-3-(tetrahydropyran-2-yloxy)-estra-1,3,5(10)-trien**

Eine Lösung von 1,64 g 17β-Acetoxy-7α-(5-methylaminopentyl)-3-(tetrahydropyran-2-yloxy)-estra-1,3,5(10)-trien in 25 ml abs. DMF wird mit 159 mg 80%igem Natriumhydrid unter Stickstoff 2 Stunden bei Raumtemperatur gerührt. Es werden dann 1,43 g 3-Chlorpropyl-4,4,5,5,5-pentafluorpentansulfid in 7 ml abs. DMF zugetropft und anschließend 22 Stunden bei 80°C nachgerührt. Die Reaktionslösung wird dann mit Essigester verdünnt, mit Wasser gewaschen, getrocknet, eingedampft und der Rückstand an Kieselgel chromatographiert. Es werden 820 mg 17β-Acetoxy-7α-{5-[N-methyl-N-3-(4,4,5,5,5-pentafluorpentylthio)-propylamino]-pentyt}-3-(tetrahydropyran-2-yloxy-estra-1,3,5(10)-trien als Öl erhalten. $[\alpha]_D^{22}$ = +21,5° (c = 0,51% in Chloroform)

**j) 7α-{5-[N-Methyl-N-3-(4,4,5,5,5-pentafluorpentylthio)-propylamino]-pentyl}-3-(tetrahydropyran-2-yloxy)-estra-1,3,5(10)-trien-17β-ol**

Eine Lösung von 790 mg 17β-Acetoxy-7α-{5-[N-Methyl-N-3-(4,4,5,5,5-pentafluor-pentylthio)-propylamino]-pentyl}-3-(tetrahydropyran-2-yloxy)-estra-1,3,5(10)-trien in 8 ml Methanol und 3 ml THF wird mit 430 mg Kaliumcarbonat 18 Stunden bei Raumtemperatur gerührt. Die Reaktionslösung wird mit Diethylether verdünnt, mit Wasser gewaschen, getrocknet und eingedampft. Es werden 750 mg rohes 7α-{5-N-Methyl-N-3-(4,4,5,5,5-pentafluor-pentylthio)-propylamino]-pentyl}-3-(tetrahydropyran-2-yloxy)-estra-1,3,5(10)-trien-17β-ol erhalten.

**k) 7α-{5-[N-Methyl-N-3-(4,4,5,5,5-pentafluorpentylthio)-propylamino]-pentyl}-estra-1,3,5(10)-trien-3,17β-diol**

Eine Loesung von 750 mg 7α-{5-[N-Methyl-N-3-(4,4,5,5,5-pentafluorpentylthio)-propylamino]-pentyl}-3-(tetrahydropyran-2-yloxy)-estra-1,3,5(10)-trien-17β-ol in 28 ml Methanol und 2,8 ml Wasser wird mit 350 mg Oxalsäure 17 Stunden bei Raumtemperatur gerührt. Es wird dann mit Essigester verdünnt, mit Natriumhydrogencarbonatlösung und Wasser gewaschen, getrockent und eingedampft. Der Rückstand wird an Kieselgel chromatographiert und es werden 640 mg 7α-{5-[N-Methyl-N-3-(4,4,5,5,5-pentafluor-pentylthio)-propylamino]-pentyl}-estra-1,3,5(10)-trien-3,17β-diol als Öl erhalten.
$[\alpha]_D^{22}$ = +24,0° (c = 0,515% in Chloroform)

**Beispiel 2**

**17α-Methyl-7α-{5-[N-methyl-N-3-(4,4,5,5,5-pentafluorpentylthio)-propylamino]-pentyl}-estra-1,3,5(10)-trien-3,17β-diol**

**a) 7α-{5-[N-Methyl-N-3-(4,4,5,5,5-pentafluorpentylthio)-propylamino]-pentyl}-3-(tetrahydropyran-2-yloxy)-estra-1,3,5(10)-trien-17-on**

Eine Lösung von 900 mg 7α-{5-[N-Methyl-N-3-(4,4,5,5,5-pentafluorpentylthio)-propylamino]-pentyl}-3-(tetrahydropyran-2-yloxy)-estra-1,3,5(10)-trien-17β-ol wird in 30 ml Toluol und 9,6 ml Cyclohexanon mit einer Lösung von 900 mg Aluminiumisopropylat in 16 ml Toluol versetzt und 30 Minuten unter langsamen abdestillieren erhitzt. Die Raektionslösung wird dann mit Essigester verdünnt, mit 20%iger Kaliumnatriumtartratlösung gewaschen, getrocknet und

eingedampft. Der Rückstand wird an Kieselgel mit Hexan / Essigester chromatographiert. Man erhält 715 mg 7α-{5-[N-Methyl-N-3-(4,4,5,5,5-pentafluor-pentylthio)-propylamino]-pentyl}-3-(tetrahydropyran-2-yloxy)-estra-1,3,5(10)-trien-17-on als Öl.

**b) 3-Hydroxy-7α-{5-[N-methyl-N-3-(4,4,5,5,5-pentafluorpentylthio)-propylamino]-pentyl}-estra-1,3,5(10)-trien-17-on**

Eine Lösung von 710 mg 7α-{5-[N-Methyl-N-3-(4,4,5,5,5-pentafluorpentylthio)-propylamino]-pentyl}-3-(tetrahydropyran-2-yloxy)-estra-1,3,5(10)-trien-17-on wird wie unter 1k beschrieben umgestzt, aufgearbeitet und an Kieselgel mit Hexan / Essigester chromatographiert. Man erhält 650 mg 3-Hydroxy-7α-{5-[N-methyl-N-3-(4,4,5,5,5-pentafluorpentylthio)-propylamino]-pentyl}-estra-1,3,5(10)-trien-17-on als Öl.

**c) 17α-Methyl-7α-{5-[N-methyl-N-3-(4,4,5,5,5-pentafluorpentylthio)-propylamino]-pentyl}-estra-1,3,5(10)-trien-3,17β-diol**

Getrocknetes Cer(III)chlorid (1,97 g) wird in 20 ml Tetrahydrofuran 25 Minuten bei Raumtemperatur gerührt, unter Eiskühlung mit 3 ml einer 3 molaren Methylmagnesiumbromidlösung versetzt, 30 Minuten unter Eiskühlung und 10 Minuten bei Raumtemperatur gerührt. In diese Reaktionsmischung wird eine Lösung von 600 mg 3-Hydroxy-7α-{5-[N-methyl-N-3-(4,4,5,5,5-pentafluor-pentylthio)-propylamino]-pentyl}-estra-1,3,5(10)-trien-17-on in 6 ml Tetrahydrofuran getropft und 2 Stunden bei Raumtemperatur nachgerührt. Bei Eiskühlung wird mit gesättigter Ammoniumchloridlösung das überschüssige Reagenz zersetzt, mit Essigester verdünnt und mit gesättigter Ammoniumchloridlösung und Wasser gewaschen, über Natriumsulfat getrocknet und i.Vak. eingeengt und an Kieselgel mit Hexan / Essigester chromatographiert. Man erhält 350 mg 17α-Methyl-7α-{5-[N-methyl-N-3-(4,4,5,5,5-pentafluorpentylthio)-propylamino]-pentyl}-estra-1,3,5(10)-trien-3,17β-diol als Öl. $[\alpha]_D^{22}$ = +15° (c = 0.52% in Chloroform)

Beispiel 3

**17α-Ethinyl-7α-{5-[N-methyl-N-3-(4,4,5,5,5-pentafluorpentylthio)-propylamino]-pentyl}-estra-1,3,5(10)-trien-3,17β-diol**

**a) 17α-Ethinyl-7α-{5-[N-methyl-N-3-(4,4,5,5,5-pentafluorpentylthio)-propylamino]-pentyl}-3-(tetrahydropyran-2-yloxy)-estra-1,3,5(10)-trien-17β-ol**

In eine Lösung von 336 mg Kalium-*tert.*-butylat in 30 ml Tetrahydrofuran wird unter Eiskühlung trockenes Acetylen bis zur Sättigung eingeleitet. In diese Lösung werden 410mg 7α-{5-[N-Methyl-N-3-(4,4,5,5,5-pentafluorpentylthio)-propylamino]-pentyl }-3-(tetrahydropyran-2-yloxy)-estra-1,3,5(10)-trien-17-on in 10 ml Tetrahydrofuran getropft und eine Stunde bei Eisbadtemperatur gerührt. Dann wird mit Essigester verdünnt, mit Wasser gewaschen, über Natriumsulfat getrocknet und i.Vak. eingeengt. Man erhält 440 mg rohes 17α-Ethinyl-7α-{5-[N-methyl-N-3-(4,4,5,5,5-pentafluorpentylthio)-propylamino]-pentyl}-3-(tetrahydropyran-2-yloxy)-estra-1,3,5(10)-trien-17β-ol.

**b) 17α-Ethinyl-7α-{5-[N-methyl-N-3-(4,4,5,5,5-pentafluorpentylthio)-propylamino]-pentyl}-estra-1,3,5(10)-trien-3,17β-diol**

Wie im Beispiel 1k beschrieben werden 440 mg rohes 17α-Ethinyl-7α-{5-[N-methyl-N-3-(4,4,5,5,5-pentafluorpentylthio)-propylamino]-pentyl}-3-(tetrahydropyran-2-yloxy)-estra-1,3,5(10)-trien-17β-ol umgestzt, aufgearbeitet und an Kieselgel mit Hexan / Essigester chromatographiert. Man erhält 275 mg 17α-Ethinyl-7α-{5-[N-methyl-N-3-(4,4,5,5,5-pentafluorpentylthio)-propylamino]-pentyl}-estra-1,3,5(10)-trien-3,17β-diol als Öl. $[\alpha]_D^{22}$ = +3,2° (c = 0,52% in Chloroform)

Beispiel 4

**7α-{5-[N-Methyl-N-2-(4,4,5,5,5-pentafluorpentansulfonyl)-ethylamino]-pentyl}-estra-1,3,5(10)-trien-3,17β-diol**

**a) 7α-{5-Methylaminopentyl}-3-(tetrahydropyran-2-yloxy)-estra-1,3,5(10)-trien-17β-ol**

Eine Lösung von 307 mg 17β-Acetoxy-7α-{5-methylaminopentyl}-3-(tetrahydropyran-2-yloxy)-estra-1,3,5 (10)-trien (Bespiel 1h) in 6 ml Methanol wird mit 1,5 ml Natronlauge 4 Stunden bei Raumtemperatur gerührt. Dann wird mit Essigester verdünnt, mit Wasser gewaschen, über Natriumsulfat getrocknet und i.Vak. eingeengt. Man erhält

285 mg 7α-{5-Methylaminopentyl}-3-(tetrahydropyran-2-yloxy)-estra-1,3,5(10)-trien-17β-ol.

**b) 7α-{5-[N-Methyl-N-2-(4,4,5,5,5-pentafluorpentansulfonyl)-ethylamino]-pentyl}-3-(tetrahydropyran-2-yloxy)-estra-1,3,5(10)-trien-17β-ol**

Eine Lösung von 270 mg 7α-{5-Methylaminopentyl}-3-(tetrahydropyran-2-yloxy)-estra-1,3,5(10)-trien-17β-ol in 9,5 ml Methanol wird mit 250 mg Vinyl-4,4,5,5,5-pentafluor-pentansulfonansulfon 23 Stunden bei 60 °C gerührt. Dann wird mit Essigester verdünnt, mit Wasser gewaschen, über Natriumsulfat getrocknet, i.Vak. eingeengt und an Kieselgel mit Hexan / Essigester chromatographiert. Man erhält 285 mg 7α-{5-[N-Methyl-N-2-(4,4,5,5,5-pentafluorpentansulfonyl)-ethylamino]-pentyl}-3-(tetrahydropyran-2-yloxy)-estra-1,3,5(10)-trien-17β-ol als Öl.

**c) 7α-{5-[N-Methyl-N-2-(4,4,5,5,5-pentafluorpentansulfonyl)-ethylamino]-pentyl}-estra-1,3,5(10)-trien-3,17β-diol**

Eine Lösung von 275 mg 7α-{5-[N-Methyl-N-2-(4,4,5,5,5-pentafluorpentansulfonyl)-ethylamino]-pentyl}-3-(tetrahydropyran-2-yloxy)-estra-1,3,5(10)-trien-17β-ol wird wie im Beispiel 1k beschrieben umgesetzt, aufgearbeitet und an Kieselgel mit Hexan / Essigester chromatographiert. Man erhält 250 mg 7α-{5-[N-Methyl-N-2-(4,4,5,5,5-pentafluor-pentansulfonyl)-ethylamino]-pentyl}-estra-1,3,5(10)-trien-3,17β-diol.
$[\alpha]_D^{22}$ = +23,6° (c = 0.52% in Chloroform)

**Beispiel 5**

**7α-{5-[N-Methyl-N-3-(4,4,5,5,5-pentafluorpentansulfonyl)-propylamino]-pentyl}-estra-1,3,5(10)-trien-3,17β-diol**

**a) 17β-Acetoxy-7α-{5-[N-methyl-N-3-(4,4,5,5,5-pentafluorpentansulfonyl)-propylamino]-pentyl}-3-(tetrahydropyran-2-yloxy)-estra-1,3,5(10)-trien**

Eine Lösung von 400 mg 17β-Acetoxy-7α-{5-methylaminopentyl}-3-(tetrahydropyran-2-yloxy)-estra-1,3,5(10)-trien (Bespiel 1h) wird wie im Beispiel 1i beschrieben mit 485 mg 3-Chlorpropyl-4,4,5,5,5-pentafluor-pentansulfon umgesetzt, aufgearbeitet und an Kieselgel mit Hexan / Essigester chromatographiert. Man erhält 190 mg 17ß-Acetoxy-7α-{5-[N-methyl-N-3-(4,4,5,5,5-pentafluorpentansulfonyl)-propylamino]-pentyl}-3-(tetrahydropyran-2-yloxy)-estra-1,3,5(10)-trien als Öl.

**b) 7α-{5-[N-Methyl-N-3-(4,4,5,5,5-pentafluorpentansulfonyl)-propylamino]-pentyl}-3-(tetrahydropyran-2-yloxy)-estra-1,3,5(10)-trien-3-ol**

Eine Lösung von 190 mg 17β-Acetoxy-7α-{5-[N-methyl-N-3-(4,4,5,5,5-pentafluor-pentansulfonyl)-propylamino]-pentyl}-3-(tetrahydropyran-2-yloxy)-estra-1,3,5(10)-trien wird wie im Beispiel 1k beschrieben umgesetzt, aufgearbeitet und an Kieselgel mit Hexan / Essigester chromatographiert. Man erhält 95 mg 17β-Acetoxy-7α-{5-[N-methyl-N-3-(4,4,5,5,5-pentafluorpentansulfonyl)-propylamino]-pentyl}-estra-1,3,5(10)-trien-3-ol als Öl.

**c) 7α-{5-[N-Methyl-N-3-(4,4,5,5,5-pentafluorpentansulfonyl)-propylamino]pentyl}-estra-1,3,5(10)-trien-3,17β-diol**

Eine Lösung von 95 mg 17β-Acetoxy-7α-{5-[N-methyl-N-3-(4,4,5,5,5-pentafluor-pentansulfonyl)-propylamino]-pentyl}-estra-1,3,5(10)-trien-3-ol wird wie im Beispiel 1i beschrieben umgesetzt, aufgearbeitet und an Kieselgel mit Hexan / Essigester chromatographiert. Man erhält 55 mg 7α-{5-[N-Methyl-N-3-(4,4,5,5,5-pentafluor-pentansulfonyl)-propylamino]-pentyl }-estra-1,3,5(10)-trien-3,17β-diol

**Beispiel 6**

**7α-{5-[N-Methyl-N-3-(4,4,5,5,5-pentafluorpentansulfinyl)-propylamino]-pentyl}-estra-1,3,5(10)-trien-3,17β-diol**

Eine Lösung von 200 mg 7α-{5-[N-Methyl-N-3-(4,4,5,5,5-pentafluorpentylthio)-propylamino]-pentyl}-estra-1,3,5(10)-trien-3,17β-diol in 8,1 ml Methanol und 1,7 ml Wasser wird mit 101 mg Natriumperiodat 2 Stunden bei Raumtemperatur gerührt. Es wird mit Essigester verdünnt, mit Wasser gewaschen, getrocknet und eingedampft. Der Rückstand wird an Kieselgel chromatographiert und es werden 150 mg 7α-{5-[N-Methyl-N-3-(4,4,5,5,5-pentafluor-pentansulfinyl)-propylamino]-pentyl}-estra-1,3,5(10)-trien-3,17β-diol als Öl erhalten.

**Beispiel 7**

**7α-{5-[N-Methyl-N-4-(4,4,5,5,5-pentafluorpentylthio)-butylamino]-pentyl}-estra-1,3,5(10)-trien-3,17β-diol**

**a) 17β-Acetoxy-7α-{5-[N-Methyl-N-4-(4,4,5,5,5-pentafluorpentylthio)-3-(tetrahydropyran-2-yloxy)-butylamino]-pentyl}-estra-1,3,5(10)-trien**

Eine Lösung von 835 mg 17β-Acetoxy-7α-(5-methylaminopentyl)-3-(tetrahydropyran-2-yloxy)-estra-1,3,5(10)-trien (Beisp. 1h) wird wie im Beispiel 1i beschieben mit 969 mg 4-Chlorbutyl-4,4,5,5,5-pentafluorpentylsulfid umgesetzt, aufgearbeitet und an Kieselgel mit Hexan / Essigester chromatographiert. Man erhält 675 mg 17β-Acetoxy-7α-{5-[N-Methyl-N-4-(4,4,5,5,5-pentafluorpentylthio)-3-(tetrahydropyran-2-yloxy)-butylami no]-pentyl }-estra-1,3,5(10)-trien als Öl.

**b) 7α-{5-[N-Methyl-N-4-(4,4,5,5,5-pentafluorpentylthio)-3-(tetrahydropyran-2-yloxy)-butylamino]-pentyl}-estra-1,3,5(10)-trien-3-ol**

Eine Lösung von 660 mg 17β-Acetoxy-7α-{5-[N-Methyl-N-4-(4,4,5,5,5-pentafluorpentylthio)-3-(tetrahydropylan-2-yloxy)-butylamino]-pentyl}-estra-1,3,5(10)-trien wird wie im Beispiel 1j beschieben umgesetzt, aufgearbeitet und an Kieselgel mit Dichlormethan / Methanol chromatographiert. Man erhält 530 mg 7α-{5-[N-Methyl-N-4-(4,4,5,5,5-pentafluorpentylthio)-3-(tetrahydropyran-2-yloxy)-butylamino]-pentyl}-estra-1,3,5(10)-trien-3-ol als Öl.

**c) 7α-{5-[N-Methyl-N-4-(4,4,5,5,5-pentafluorpentylthio)-butylamino]-pentyl}-estra-1,3,5(10)-trien-3,17β-diol**

Eine Lösung von 530 mg 7α-{5-[N-Methyl-N-4-(4,4,5,5,5-pentafluorpentylthio)-3-(tetrahydropyran-2-yloxy)-butylamino]-pentyl}-estra-1,3,5(10)-trien-3-ol wird wie im Beispiel 1k beschieben umgesetzt, aufgearbeitet und an Kieselgel mit Dichlormethan / Methanol chromatographiert. Man erhält 470 mg 7α-{5-[N-Methyl-N-4-(4,4,5,5,5-pentafluorpentylthio)-butylamino]-pentyl}-estra-1,3,5(10)-trien-3,17β-diol als Öl. $[\alpha]_D^{22}$ = +20,5° (c = 0.515% in Chloroform).

**Beispiel 8**

**2-Fluor-7α-{5-[N-Methyl-N-3-(4,4,5,5,5-pentafluorpentylthio)-propylamino]-pentyl}-estra-1,3,5(10)-trien-3,17β-diol**

**a) 7α-(5-*tert.* Butyl-dimethylsilyloxypentyl)-estr-4-en-3,17-dion**

In 70 ml abs. Tetrahydrofuran werden 15,1 g Mg-Späne mit 175,6 g 1-Brom-5-*tert*-butyl-dimethylsilyloxypentan [Tetrahedron Letters 23, **1982,** 40, 4147-4150] gelöst in 600 ml abs. Tetrahydrofuran zum Grignardreagenz umgesetzt. In diese auf -20 °C gekühlte Lösung werden unter einem Stickstoffstrom 59 g Kupfer-(I)-iodid gegeben und anschließend innerhalb einer Stunde 50 g Estra-4,6-dien-3,17-dion [Steroids Vol. 1, **1963,** 233-249] gelöst in 300 ml abs. THF zugetropft. Zur Aufarbeitung werden 37,5 ml Essigsäure zugetropft, die Reaktionsmischung mit Essigester verdünnt, mit gesättigter Ammoniumchloridlösung, Wasser und Natriumhydrogencarbonatlösung gewaschen und getrocknet. Der nach dem Eindampfen erhaltene Rückstand wird an Kieselgel chromatographiert. Man erhält 35,4 g 7α-(5-*tert*-Butyl-dimethylsilyloxypentyl)-estr-4-en-3,17-dion. $[\alpha]_D^{22}$ = +52.8° (c = 0.535% in Chloroform).

**b) 7α-(5-Hydroxypentyl)-estr-4-en-3,17-dion**

Eine Lösung von 125,4 g 7α-(5-*tert*-Butyl-dimethylsilyloxypentyl)-estr-4-en-3,17-dion in 625 ml Methanol und 347 ml Wasser wird mit 694 ml Eisessig 2,5 Stunden bei 50 °C gerührt. Nach dem Eindampfen bei 60 °C i. Vak. werden 94,1 g rohes 7α-(5-Hydroxypentyl)-estr-4-en-3,17-dion als Öl erhalten.

**c) 7α-(5-Acetoxypentyl)-estr-4-en-3,17-dion**

Eine Lösung von 94 g rohem 7a-(5-Hydroxypentyl)-estr-4-en-3,17-dion in 620 ml Pyridin wird mit 310 ml Acetanhydrid langsam versetzt und 2 h bei 25 °C gerührt. Dann wird unter Eiskühlung mit 116 ml Wasser langsam versetzt, mit 3 l Diethylether verdünnt, die organ.
Phase nach dem Waschen mit Natriumhydrogencarbonatlösung, getrocknet und eingedampft. Der Rückstand wird an Kieselgel chromatographiert und es werden 84,4 g 7α-(5-Acetoxypentyl)-estr-4-en-3,17-dion als Öl erhalten.

**d) 7α-(5-Acetoxypentyl)-3-hydroxy-estra-1,3,5(10)-trien-17-on**

Zu einer Lösung von 82,3 g 7a-(5-Acetoxypentyl)-estr-4-en-3,17-dion in 936 ml Acetonitril werden bei 80 °C Badtemperatur 17,8 g Lithiumbromid und 92,83 g Kupfer-II-bromid gegeben. Nach 10 Minuten bei 80 °C Badtemperatur wird die Reaktionslösung abgekühlt, mit Wasser versetzt, dreimal mit Essigester extrahiert, die organische Phase mit Wasser und Natriumhydrogencarbonatlösung gewaschen und getrocknet. Der nach dem Eindampfen erhaltene Rückstand wird an Kieselgel chromatographiert und es werden 60,4 g 7α-(5-Acetoxypentyl)-3-hydroxy-estra-1,3,5(10)-trien-17-on als Öl erhalten.

**e) 7α-(5-Acetoxypentyl)-estra-1,3,5(10)-trien-3,17β-diol**

In 65 ml Tetrahydrofuran löst man 6,0 g 7α-(5-Acetoxypentyl)-3-hydroxy-estra-1,3,5(10)-trien-17-on und gibt bei Raumtemperatur 2 ml Wasser und 0,6 g feines Natriumborhydrid zu. Nach 1 Stunde rühren bei Raumtemperatur wird die Reaktionsmischung in Eiswasser gegeben, dreimal mit Essigester extrahiert und die organische Phase mit Natriumchloridlösung gewaschen, mit Natriumsulfat getrocknet und im Vakuum eingedampft, Rohausbeute 6,07 g.

**f) 7α-(5-Acetoxypentyl)-3,17β-bis-(tetrahydropyran-2-yloxy)-estra-1,3,5(10)-trien**

5,6 g rohes 7α-(5-Acetoxypentyl)-estra-1,3,5(10)-trien-3,17β-diol werden in 35 ml wasserfreiem Tetrahydrofuran gelöst und mit 3,5 ml Dihydropyran und 0,18 g p-Toluolsulfonsäure versetzt. Man rührt die Mischung bei Raumtemperatur über Nacht, verdünnt dann mit Essigester und schüttelt die Mischung nacheinander mit gesättigter Natriumbicarbonatlösung und gesättigter Kochsalzlösung aus. Die organische Phase wird mit Natriumsulfat getrocknet und im Vakuum eindegampft, Ausbeute 8,31 g

**g) 7α-(5-Benzolsulfonyloxypentyl)-3,17β-bis-(tetrahydropyran-2-yloxy)-2-fluorestra-1,3,5(10)-trien und 7α-(5-Benzolsulfonyloxypentyl)-17β-(tetrahydropyran-2-yloxy)-2-fluor-estra-1,3,5(10)-trien-3-ol**

Man löst 8,8 g des nicht weiter aufgereinigten 7a-(5-Acetoxypentyl)-3,17β-bis-(tetrahydro-pyran-2-yloxy)-estra-1,3,5(10)-triens in 220 ml wasserfreiem Tetrahydrofuran. In einer Inertgasatmosphäre kühlt man auf -70 °C ab und versetzt mit 88 ml einer etwa 1,3 molaren Lösung von sec.-Butyllithium in Hexan. Man rührt anschließend 30 Minuten bei -70 °C und 30 Minuten bei 0 °C, kühlt wieder auf -70 °C ab und tropft die Lösung von 34,68 g N-Fluorbenzolsulfonimid in 280 ml wasserfreiem Tetrahydrofuran zu. Wieder wird 30 Minuten bei -70 °C und 30 Minuten bei 0 °C gerührt, dann in eine gesättigte Natriumbicarbonatlösung gegoßen und dreimal mit Essigester extrahiert. Die vereinigten organischen Phasen werden mit gesättigter Kochsalzlösung ausgeschüttelt, mit Natriumsulfat getrocknet und im Vakuum eindampft. Die Rohausbeute von 26 g wird zweimal an Kieselgel mit Hexan chromatographiert, wobei man 1,52 g 7α-(5-Benzolsulfonyloxypentyl)-3,17β-bis-(tetrahydropyran-2-yloxy)-2-fluor-estra-1,3,5(10)-trien als Öl und 210 mg 7α-(5-Benzolsulfonyloxypentyl)-17β-(tetrahydropyran-2-yloxy)-2-fluor-estra-1,3,5(10)-trien-3-ol als Öl erhält.

**h) 2-Fluor-7α-{5-[N-Methyl-N-3-(4,4,5,5,5-pentafluorpentylthio)-propylamino]-pentyl}-17β-(tetrahydropyran-2-yloxy)-estra-1,3,5(10)-trien-3-ol**

In eine Lösung von 210 mg des oben erhaltenen 7α-(5-Benzolsulfonyloxypentyl)-17β-(tetrahydropyran-2-yloxy)-2-fluor-estra-1,3,5(10)-trien-3-ols in 1,8 ml wasserfreiem Dimethylformamid, gibt 130 mg Methyl-[3-(4,4,5,5,5-pentafluorpentylthio)-propyl]-amin zu und erhitzt in einer Inertgasatmosphäre 6 Stunden auf 100 °C. Nach dem Abkühlen wird wieder mit Essigester verdünnt, mit Natriumhydrogencarbonatlösung versetzt und die wässrige Phase zweimal mit Essigester, die vereinigten organischen Phasen mit gesättigter Kochsalzlösung ausgeschüttelt und die organische Phase mit Natriumsulfat getrocknet und im Vakuum vom Solvens befreit. Der Rückstand von 292 mg wird an Kieselgel mit Essigester und Methanol chromatographiert und man erhält 105 mg 2-Fluor-7α-{5-[N-Methyl-N-3-(4,4,5,5,5-pentafluorpentylthio)-propylamino]-pentyl}-17β-(tetrahydropyran-2-yloxy)-estra-1,3,5(10)-trien-3-ol als Öl.

**i) 2-Fluor-7α-{5-[N-Methyl-N-3-(4,4,5,5,5-pentafluorpentylthio)-propylamino]-pentyl}-estra-1,3,5(10)-trien-3,17β-diol**

In 3 ml Methanol und 0,3 ml Wasser löst man die erhaltenen 105 mg 2-Fluor-7α-{5-[N-Methyl-N-3-(4,4,5,5,5-pentafluorpentylthio)-propylamino]-pentyl}-17β-(tetrahydropyran-2-yloxy)-estra-1,3,5(10)-trien-3-ol und 60 mg Oxalsäure, erwärmt 45 Minuten auf 60 °C, versetzt nach dem Abkühlen mit Essigester und verteilt wie oben beschrieben zwischen gesättigter Natriumbicarbonatlösung und Essigester. Die organische Phase wird mit Natriumchloridlösung gewaschen, in Natriumsulfat getrocknet und im Vakuum eingedampft. Durch präparative Dünnschichtchromatographie an Kieselgel mit Essigester/Methanol 9:1 als Laufmittel erhält man 46 mg 2-Fluor-7α-{5-[N-Methyl-N-3-(4,4,5,5,5-pentafluorpentylthio)-propylamino]-pentyl}-estra-1,3,5(10)-trien-3,17β-diol als Öl mit einem Drehwert von $[\alpha]^{22}_{D}$ = +24,0° (c = 0.1% in Chloroform).

**Beispiel 9**

**2-Fluor-17α-methyl-7α-{5-[N-Methyl-N-3-(4,4,5,5,5-pentafluorpentylthio)-propylamino]-pentyl}-estra-1,3,5(10)-trien-3,17β-diol**

**a) 2-Fluor-7α-{5-[N-Methyl-N-3-(4,4,5,5,5-pentafluorpentylthio)-propylamino]-pentyl}-estra-1,3,5(10)-trien-3-ol-17-on**

Aus 83 mg 2-Fluor-7α-{5-[N-Methyl-N-3-(4,4,5,5,5-pentafluorpentylthio)-propylamino]-pentyl}-estra-1,3,5(10)-trien-3,17β-diol erhält man wie in Beispiel 2a) beschrieben 70 mg 2-Fluor-7α-{5-[N-Methyl-3-(4,4,5,5,5-pentafluorpentylthio)-propoylamino]-pentyl}-estra-1,3,5(10)-trien-3-ol-17-on als Öl.

**b) 2-Fluor-17α-methyl-7α-{5-[N-Methyl-N-3-(4,4,5,5,5-pentafluorpentylthio)-propylamino]-pentyl}-estra-1,3,5(10)-trien-3,17β-diol**

Wie im Beispiel 2c) beschrieben erhält man aus 65 mg 2-Fluor-7α-{5-[N-Methyl-N-3-(4,4,5,5,5-pentafluorpentylthio)-propylamino]-pentyl}-estra-1,3,5(10)-trien-3-ol-17-on 32 mg 2-Fluor-17α-methyl-7α-{5-[N-Methyl-N-3-(4,4,5,5,5-pentafluorpentylthio)-propylamino]-pentyl}-estra-1,3,5(10)- trien-3,17β-diol als Öl.

**Beispiel 10**

**2-Fluor-7α-{5-(N-Methyl-3-(4,4,5,5,5-pentafluorpentansulfinyl)-propylamino]-pentyl}-estra-1,3,5(10)-trien-3,17β-diol**

Wie in Beispiel 1 erhält man durch Oxidation mit Natriumperiodat aus 75 mg 2-Fluor-7α-{5-[N-Methyl-N-3-(4,4,5,5,5-pentafluorpentylthio)-propylamino]-pentyl}-estra-1,3,5(10)-trien-3,17β-diol 44 mg 2-Fluor-7α-{5-[N-Methyl-3-(4,4,5,5,5-pentafluorpentansulfinyl)-propylamino]-pentyl}-estra-1,3,5(10)-trien-3,17β-diol als festen Schaum.

**Beispiel 11**

**2-Fluor-17α-methyl-7α-{5-[N-Methyl-N-3-(4,4,5,5,5-pentafluorpentansulfonyl)-propylamino]-pentyl}-estra-1,3,5(10)-trien-3,17β-diol**

**a) 3,17β-Bis-(tetrahydropyran-2-yloxy)-2-fluor-7α-{5-[N-Methyl-N-3-(4,4,5,5,5-pentafluorpentansulfonyl)-propylamino]-pentyl}-estra-1,3,5(10)-trien**

Man setzt 283 mg 7α-(5-Benzolsulfonyloxypentyl)-3,17β-bis-(tetrahydropyran-2-yloxy)-2-fluor-estra-1,3,5(10)-trien aus Beispiel 6g) wie im Beispiel 6h) beschrieben mit 250 mg Methyl-[3-(4,4,5,5,5-pentafluorpentansulfonyl)-propyl]-amin um und erhält 180 mg 3,17β-Bis-(tetrahydropyran-2-yloxy)-2-fluor-7α-{5-[N-Methyl-N-3-(4,4,5,5,5-pentafluorpentansulfonyl)-propylamino]-pentyl}-estra-1,3,5(10)-trien als Öl.

**b) 2-Fluor-7α-{5-[N-Methyl-N-3-(4,4,5,5,5-pentafluorpentansulfonyl)-propylamino]-pentyl}-estra-1,3,5(10)-trien-3,17β-diol**

Zur Spaltung der THP-Ether mit Oxalsäure löst man die 180 mg 3,17β-Bis-(tetrahydropyran-2-yloxy)-2-fluor-7α-{5-(N-Methyl-N-3-(4,4,5,5,5-pentafluorpentansulfonyl)-propy lamino]-pentyl}-astra-1,3,5(10)-trien in Methanol und Wasser und erhält wie im Beispiel 6i) beschrieben 123 mg 2-Fluor-7α-{5-[N-Methyl-N-3-(4,4,5,5,5-pentafluorpentansulfonyl)-propylamino]-pentyl}-estra-1,3,5(10)-trien-3,17β-diol als festen Schaum.

**c) 2-Fluor-7α-{5-[N-Methyl-N-3-(4,4,5,5,5-pentafluorpentansulfonyl)-propylamino]-pentyl}-estra-1,3,5(10)-trien-3-ol-17-on**

Wie in Beispiel 2a) beschrieben werden 70 mg 2-Fluor-7α-{5-[N-Methyl-N-3-(4,4,5,5,5-pentafluorpentansulfonyl)-propylamino]-pentyl}-estra-1,3,5(10)-trien-3,17β-diol oxidiert, wobei man 52 mg 2-Fluor-7α-{5-[N-Methyl-N-3-(4,4,5,5,5-pentafluorpentansulfonyl)-propylamino]-pentyl}-estra-1,3,5(10)-trien-3-ol-17-on erhält.

**d) 2-Fluor-17α-methyl-7α-{5-[N-Methyl-N-3-(4,4,5,5,5-pentafluorpentansulfonyl)-propylamino]-pentyl}-estra-1,3,5(10)-trien-3,17β-diol**

Wie im Beispiel 2c) beschrieben erhält man aus 45 mg 2-Fluor-7α-{5-[N-Methyl-N-3-(4,4,5,5,5-pentafluorpentansulfonyl)-propylamino]-pentyl}-estra-1,3,5(10)-trien-3-ol-17-on durch Grignardreaktion 38 mg 2-Fluor-17α-methyl-7α-{5-[N-Methyl-N-3-(4,4,5,5,5-pentafluorpentansulfonyl)-propylamino]-pentyl}-estra-1,3,5(10)-trien-3,17β-diol als festen Schaum.

**Beispiel 12**

**7α-{5-[N-Methyl-N-4-(4,4,5,5,5-pentafluorpentansulfinyl)-butylamino]-pentyl}-estra-1,3,5(10)-trien-3,17β-diol**

Eine Lösung von 150 mg 7α-{5-[N-Methyl-N-4-(4,4,5,5,5-pentafluorpentylthio)-butylamino]-pentyl}-estra-1,3,5(10)-trien-3,17β-diol wird wie im Beispiel 6 beschrieben umgesetzt, aufgearbeitet und an Kieselgel mit Dichlormethan / Methanol chromatographiert. Man erhält 140 mg 7α-{5-[N-Methyl-N-4-(4,4,5,5,5-pentafluorpentansulfinyl)-butylamino]-pentyl}-estra-1,3,5(10)-trien-3,17b-diol als festen Schaum. $[\alpha]_D^{22}$ = +22,9° (c = 0.505% in Methanol).

Beispiel 13

**17α-Methyl-7α-{5-[N-methyl-N-3-(4,4,5,5,5-pentafluorpentansulfinyl)-propylamino]-pentyl}-estra-1,3,5(10)-trien-3,17β-diol**

Eine Lösung von 390 mg 17α-Methyl-7α-{5-[N-methyl-N-3-(4,4,5,5,5-pentafluorpentylthio)-propylamino]-pentyl}-estra-1,3,5(10)-trien-3,17β-diol wird wie im Beispiel 6 beschrieben umgesetzt, aufgearbeitet und an Kieselgel mit Dichlormethan / Methanol chromatographiert. Man erhält 260 mg 17α-Methyl-7α-{5-[N-methyl-N-3-(4,4,5,5,5-pentafluorpentansulfinyl)-propylamino]-pentyl}-estra-1,3,5(10)-trien-3,17β-diol als Schaum. $[\alpha]_D^{22}$ = +1,3° (c = 0.51% in Chloroform).

**Beispiel 14**

**17α-Methyl-7α-{5-[N-methyl-N-3-(4,4,5,5,5-pentafluorpentansulfonyl)-propylamino]-pentyl}-estra-1,3,5(10)-trien-3,17β-diol**

**a) 7α-(5-*tert*. Butyl-dimethylsilyloxypentyl)-estr-4-en-3,17-dion**

In 70 ml abs. Tetrahydrofuran werden 15,1 g Mg-Späne mit 175,6 g 1-Brom-5-*tert*-butyl-dimethylsilyloxypentan [Tetrahedron Letters 23, **1982,** 40, 4147-4150] gelöst in 600 ml abs. Tetrahydrofuran zum Grignardreagenz umgesetzt. In diese auf -20°C gekühlte Lösung werden unter einem Stickstoffstrom 59 g Kupfer-(I)-iodid gegeben und anschließend innerhalb einer Stunde 50 g Estra-4,6-dien-3,17-dion [Steroids Vol. 1, **1963**, 233-249] gelöst in 300 ml abs. THF zugetropft. Zur Aufarbeitung werden 37,5 ml Essigsäure zugetropft, die Reaktionsmischung mit Essigester verdünnt, mit gesättigter Ammoniumchloridlösung, Wasser und Natriumhydrogencarbonatlösung gewaschen und getrocknet. Der nach dem Eindampfen erhaltene Rückstand wird an Kieselgel chromatographiert. Man erhält 35,4 g 7α-(5-*tert*-Butyl-dimethylsilyloxypentyl)-estr-4-en-3,17-dion. $[\alpha]_D^{22}$ = +52.8° (c = 0.535% in Chloroform)

**b) 7α-(5-Hydroxypentyl)-estr-4-en-3,17-dion**

Eine Lösung von 125,4 g 7α-(5-*tert*-Butyl-dimethylsilyloxypentyl)-estr-4-en-3,17-dion in 625 ml Methanol und 347 ml Wasser wird mit 694 ml Eisessig 2,5 Stunden bei 50 °C gerührt.
Nach dem Eindampfen bei 60 °C i. Vak. werden 94,1 g rohes 7α-(5-Hydroxypentyl)-estr-4-en-3,17-dion als Öl erhalten.

**c) 17β-Acetoxy-7α-(5-acetoxypentyl)-estr-4-en-3,17-dion**

Eine Lösung von 94 g rohem 7α-(5-Hydroxypentyl)-estr-4-en-3,17-dion in 620 ml Pyridin wird mit 310 ml Acetanhydrid langsam versetzt und 2 h bei 25 °C gerührt. Dann wird unter Eiskühlung mit 116 ml Wasser langsam versetzt, mit 3 1 Diethylether verdünnt, die organ. Phase nach dem Waschen mit Natruimhydrogencarbonatlösung,

getrocknet und eingedampft. Der Rückstand wird an Kieselgel chromatographiert und es werden 84,4 g 7α-(5-Acetoxypentyl)-estr-4-en-3,17-dion als Öl erhalten.

**d) 7α-(5-Acetoxypentyl)-3-hydroxy-estra-1,3,5(10)-trien-17-on**

Zu einer Lösung von 82,3 g 7α-(5-Acetoxypentyl)-estr-4-en-3,17-dion in 936 ml Acetonitril werden bei 80 °C Badtemperatur 17,8 g Lithiumbromid und 92,83 g Kupfer-II-bromid gegeben. Nach 10 Minuten bei 80 °C Badtemperatur wird die Reaktionslösung abgekühlt, dreimal mit Essigester extrahiert, mit Wasser und Natriumhydrogencarbonatlösung gewaschen und getrocknet. Der nach dem Eindampfen erhaltene Rückstand wird an Kieselgel chromatographiert und es werden 60,4 g 7α-(5-Acetoxypentyl)-3-hydroxy-estra-1,3,5(10)-trien-17-on als Öl erhalten.

**e) 7α-(5-Acetoxypentyl)-3-(tetrahydropyran-2-yloxy)-estra-1,3,5(10)-trien-17-on**

Eine Lösung von 25,1 g 7α-(5-Acetoxypentyl)-3-hydroxy-estra-1,3,5(10)-trien-17-on wird wie im Beispiel le beschrieben umgesetzt, aufgearbeitet und an Kieselgel mit Hexan / Essigester chromatographiert. Man erhält 28,3 g 7α-(5-Acetoxypentyl)-3-(tetrahydropyran-2-yloxy)-estra-1,3,5(10)-trien-17-on als Öl.

**f) 3-(Tetrahydropyran-2-yloxy)-7α-(5-*p*-tosyloxypentyl)-estra-1,3,5(10)-trien-17-on**

Eine Lösung von 19,7 g 7α-(5-Acetoxypentyl)-3-(tetrahydropyran-2-yloxy)-estra-1,3,5(10)-trien-17-on wird wie im Beispiel 1f beschrieben verseift und nach Beispiel 1g tosyliert und aufgearbeitet. Man erhält 14,5 g 3-(Tetrahydropyran-2-yloxy)-7α-(5-*p*-tosyloxypentyl)-estra-1,3,5(10)-trien-17-on als Öl.

**g) 7α-{5-[N-Methyl-N-3-(4,4,5,5,5-pentafluorpentansulfonyl)-propylamino]-pentyl}-3-(tetrahydropyran-2-yloxy)-estra-1,3,5(10)-trien-17-on**

Eine Lösung von 1,5 g 3-(Tetrahydropyran-2-yloxy)-7α-(5-tosyloxypentyl)-estra-1,3,5(10)-trien-17-on in 21 ml Ethylmethylketon wird mit 716 mg Kaliumcarbonat, 171 mg Kaliumjodid und 1,5 g Methyl-[3-(4,4,5,5,5-pentafluorpentansulfonyl)-propyl]-amin 4 Stunden bei 80 °C unter Stickstoff gerührt. Dann wird mit Essigester verdünnt, mit Wasser gewaschen, über Natriumsulfat getrocknet, i.Vak. eingeengt und an Kieselgel mit Dichlormethan / Methanol chromatographiert. Man erhält 1,39 g 7α-{5-[N-Methyl-N-3-(4,4,5,5,5-pentafluor-pentansulfonyl)-propylamino]-pentyl}-3-(tetrahydropyran-2-yloxy)-estra-1,3,5(10)-trien-17-on als Öl.

**h) 17α-Methyl-7α-{5-[N-methyl-N-3-(4,4,5,5,5-pentafluorpentansulfonyl)-propylamino]-pentyl}-3-(tetrahydropyran-2-yloxy)-estra-1,3,5(10)-trien-17β-ol**

Wie im Beispiel 2c beschrieben werden 690 mg 7α-{5-[N-Methyl-N-3-(4,4,5,5,5-pentafluor-pentansulfonyl)-propylamino]-pentyl}-3-(tetrahydropyran-2-yloxy)-estra-1,3,5(10)-trien-17-on umgesetzt, aufgearbeitet und an Kieselgel mit Dichlormethan / Methanol chromatographiert. Man erhält 460 mg 17α-Methyl-7α-{5-[N-methyl-N-3-(4,4,5,5,5-pentafluorpentansulfonyl)-propylamino]-pentyl}-3-(tetrahydropyran-2-yloxy)-estra-1,3,5(10)-trien-17β-ol

**i) 17α-Methyl-7α-{5-[N-methyl-N-3-(4,4,5,5,5-pentafluorpentansulfonyl)-propylamino]-pentyl}-estra-1,3,5(10)-trien-3,17β-diol**

Eine Lösung von 460 mg 17α-Methyl-7α-{5-[N-methyl-N-3-(4,4,5,5,5-pentafluor-pentansulfonyl)-propylamino]-pentyl}-3-(tetrahydropyran-2-yloxy)-estra-1,3,5(10)-trien-17β-ol wird wie im Beispiel 1k beschrieben umgesetzt, aufgearbeitet und an Kieselgel mit Dichlormethan / Methanol chromatographiert. Man erhält 380 mg 17α-Methyl-7α-{5-[N-methyl-N-3-(4,4,5,5,5-pentafluorpentansulfonyl)-propylamino]-pentyl}-estra-1,3,5(10)-trien-3,17β-diol als festen Schaum. $[\alpha]_D^{22}$ = +12,3° (c = 0.525% in Chloroform).

**Beispiel 15**

**17α-Ethinyl-7α-{5-[N-methyl-N-3-(4,4,5,5,5-pentafluorpentansulfonyl)-propylamino]-pentyl}-estra-1,3,5(10)-trien-3,17β-diol**

**a) 17α-Ethinyl-7α-{5-[N-methyl-N-3-(4,4,5,5,5-pentafluorpentansulfonyl)-propylamino]-pentyl}-3-(tetrahydropyran-2-yloxy)-estra-1,3,5(10)-trien-17-ol**

Zu 9 ml einer eisgekühlten Tetrahydrofuran-Acetylen-Lösung werden 8,3 ml einer 3 molaren Ethylmagnesiumbromidlösung getropft. Anschließend wird eine Lösung von 660 mg 7α-{5-[N-Methyl-N-3-(4,4,5,5,5-pentafluorpentansulfonyl)-propylamino]-pentyl}-3-(tetrahydropyran-2-yloxy)-estra-1,3,5(10)-trien-17-on in 5 ml Tetrahydrofuran zugetropft und 2 Stunden bei Raumtemperatur gerührt. Die Reaktionslösung wird mit Essigester verdünnt, mit gesättigter Ammoniumchloridlösung und Wasser gewaschen, über Natriumsulfat getrocknet, i.Vak. eingeengt und an Kieselgel mit Dichlormethan / Methanol chromatographiert. Man erhält 550 mg 17α-Ethinyl-7α-{5-[N-methyl-N-3-(4,4,5,5,5-pentafluorpentansulfonyl)-propylamino]-pentyl}-3-(tetrahydropyran-2-yloxy)-estra-1,3,5(10)-trien-17-ol als Öl.

**b) 17α-Ethinyl-7α-{5-[N-methyl-N-3-(4,4,5,5,5-pentafluorpentansulfonyl)-propylamino]-pentyl}-estra-1,3,5(10)-trien-3,17β-diol**

Eine Lösung von 540 mg 17α-Ethinyl-7α-{5-[N-methyl-N-3-(4,4,5,5,5-pentafluorpentan-sulfonyl)-propylamino]-pentyl}-3-(tetrahydropyran-2-yloxy)-estra-1,3,5(10)-trien-17-ol wird wie im Beispiel 1k beschrieben umgesetzt, aufgearbeitet und an Kieselgel mit Dichlormethan / Methanol chromatographiert. Man erhält 420 mg 17α-Ethinyl-7α-{5-[N-methyl-N-3-(4,4,5,5,5-pentafluorpentansulfonyl)-propylamino]-pentyl}-estra-1,3,5(10)-trien-3,17β-diol als festen Schaum. $[\alpha]_D^{22}$ = +0,9° (c = 0.51% in Chloroform).

**Synthese der Ausgangsverbindungen**

**3-Chlorpropyl-4,4,5,5,5-pentafluorpentylsulfid**

Zu einer Mischung von 6 ml Methanol und 6 ml 30%iger methanolischer Natriummethylatlösung werden bei Eisbadtemperatur 7,08 g 4,4,5,5,5-Pentafluorpentyl-1-thioacetat zugetropft und 30 Minuten bei Raumtemperatur nachgerührt. Diese Lösung wird dann bei Raumtemperatur zu 12,2 g 1-Chlor-3-iodpropan in 12 ml Methanol gelöst, zugetropft und 3 Stunden nachgerührt. Es wird dann mit Diethylether verdünnt, mit Wasser gewaschen und getrocknet. Nach dem Eindampfen wird der Rückstand an Kieselgel chromatographiert und es werden 7,5 g 3-Chlorpropyl-4,4,5,5,5-pentafluorpentylsulfid erhalten. Eine destillierte Probe hat einen Kp. von 95-100°C bei 15 Torr.

**4-Chlorbutyl-4,4,5,5,5-pentafluorpentylsulfid**

Analog wie für das 3-Chlorpropyl-4,4,5,5,5-pentafluorpentylsulfid beschrieben erhält man 4-Chlorbutyl-4,4,5,5,5-pentafluorpentylsulfid wenn 1-Chlor-3-iodpropan durch 1-Chlor-4-iodbutan ersetzt.

**Vinyl-4,4,5,5,5-pentafluorpentylsulfon**

**a) Vinyl-4,4,5,5,5-pentafluorpentylsulfid**

Zu einer Mischung von 2,5 ml Methanol und 2 ml 30%iger methanolischer Natriummethanolatlösung werden bei Eisbadtemperatur 2,36 g 4,4,5,5,5-pentafluorpentyl-1-thioacetat getropft und 30 Minuten bei Raumtemperatur gerührt. Diese Lösung tropft man bei Raumtemperatur zu einer Lösung von 2,0 g 1-Brom-2-chlorethan in 2 ml Methanol und rührt weitere 2,5 Stunden. Dann werden 4 ml einer 30%igen Natriummethanolatlösung zugegeben und 19 Stunden rückfluxiert. Zur Aufarbeitung wird mit Diethylether verdünnt, mit Wasser gewaschen, über Natriumsulfat getrocknet, i.Vak. eingeengt und an Kieselgel mit Hexan / Essigester chromatographiert. Man erhält 290 mg Vinyl-4,4,5,5,5-pentafluorpentylsulfid.

**b) Vinyl-4,4,5,5,5-pentafluorpentylsulfon**

Eine Lösung von 290 mg Vinyl-4,4,5,5,5-pentafluorpentylsulfid in 3 ml Essigsäure wird mit 0,81 ml 30%igem Wasserstoffperoxid 19 Stunden bei Raumtemperatur gerührt. Die Reaktionslösung wird mit Diethylether verdünnt, mit Wasser gewaschen, über Natriumsulfat getrocknet, i.Vak. eingeengt und an Kieselgel mit Hexan / Essigester chromatographiert. Man erhält 250 mg Vinyl-4,4,5,5,5-pentafluorpentylsulfon vom Schmelzpunkt 28-29 °C.

**3-Chlorpropyl-4,4,5,5,5-pentafluorpentylsulfon**

Eine Lösung von 114,2 g 3-Chlorpropyl-4,4,5,5,5-pentafluorpentylsulfid in 1,1 l Chloroform wird bei 0 °C portionsweise mit 98 g 70%iger meta-Chlorperbenzoesäure versetzt und 0,5 Stunden gerührt. Die Reaktionslösung wird mit Natriumhydrogensufit-, Natriumhydrogencarbonat-, Kochsalzlösung und Wasser gewaschen, über Natriumsulfat getrocknet, i.Vak. eingeengt und an Kieselgel mit Dichlormethan / Methanol chromatographiert. Man erhält 70 g 3-Chlorpropyl-4,4,5,5,5-pentafluorpentylsulfon vom Schmelzpunkt 74-76 °C.

**Methyl-[3-(4,4,5,5,5-pentafluorpentylthio)-propyl]-amin**

**a) 3-Iodpropyl-4,4,5,5,5-pentafluorpentylsulfid**

Eine Lösung von 22,8 g 3-Chlorpropyl-4,4,5,5,5-pentafluorpentylsulfid in 500 ml Ethylmethylketon wird mit 40 g Natriumiodid 5 Stunden bei 100 °C Badtemperatur unter Stickstoff gerührt. Dann wird i. Vak. zur Trockne eingeengt, auf Wasser gegeben, dreimal mit Essigester extrahiert, neutralgewaschen, und über Natriumsulfat getrocknet und i. Vak. eingeengt. Man erhält 30,6 g 3-Iodpropyl-4,4,5,5,5-pentafluorpentylsulfid.

**b) Methyl-[3-(4,4,5,5,5-pentafluorpentylthio)-propyl]-amin**

In eine Lösung von 30,6 g 3-Iodpropyl-4,4,5,5,5-pentafluorpentylsulfid in 200 ml abs. Tetrahydrofuran werden bei -78 °C Badtemperatur 45 g Methylamin kondesiert und 1,5 Stunden bei Raumtemperatur sowie 4 Stunden bei 60 °C im Druckreaktor gerührt. Zur Öffnung des Reaktors läßt man über Nacht auf Raumtemperatur und dann auf-78 °C abkühlen. Dann läßt man auf Raumtemperatur kommen, überschüssiges Methylamin abdampfen, verdünnt mit Essigester, wäscht neutral, trocknet über Natriumsulfat, engt i.Vak. ein und chromatographiert an Kieselgel mit Dichlormethan / Methanol. Man enthält 15,7 g Methyl-[3-(4,4,5,5,5-pentafluorpentylthio)-propyl]-amin als Öl.

**3-Chlorpropyl-4,4,5,5,5-pentafluorpentylsulfon**

Eine Lösung von 5 g 3-Chlorpropyl-4,4,5,5,5-pentafluorpentylsulfid in 50 ml Essigsäure wird mit 14 ml 30%igem Wasserstoffperoxid 19 Stunden bei Raumtemperatur gerührt. Die Reaktionslösung wird mit Diethylether verdünnt, mit Wasser gewaschen, über Natriumsulfat getrocknet, i.Vak. eingeengt und an Kieselgel mit Hexan / Essigester chromatographiert. Man erhält 5,1 g 3-Chlorpropyl-4,4,5,5,5-pentafluorpentylsulfon vom Schmelzpunkt 61-62 °C.

**Methyl-[3-(4,4,5,5,5-pentafluorpentansulfonyl)-propyl]-amin**

**a) 3-Iodpropyl-4,4,5,5,5-pentafluorpentansulfon**

Eine Lösung von 23,5 g 3-Chlorpropyl-4,4,5,5,5-pentafluorpentansulfon in 500 ml Ethylmetylketon wird mit 40 g Natriumiodid 5 Stunden bei 100 °C Badtemperatur unter Stickstoff gerührt. Dann wird i. Vak. zur Trockne eingeengt, auf Wasser gegeben, dreimal mit Essigester extrahiert, neutralgewaschen, und über Natriumsulfat getrocknet und i. Vak. eingeengt. Man erhält 30,6 g 3-Iodpropyl-4,4,5,5,5-pentafluorpentansulfon als Kristalle vom Schmelzpunkt 88-89 °C.

**b) Methyl-[3-(4,4,5,5,5-pentafluorpentansulfonyl)-propyl]-amin**

In eine Lösung von 23,5 g 3-Iodpropyl-4,4,5,5,5-pentafluorpentansulfon in 200 ml abs. Tetrahydrofuran werden bei -78 °C Badtemperatur 44 g Methylamin kondensiert und 1,5 Stunden bei Raumtemperatur sowie 4 Stunden bei 60 °C im Druckreaktor gerührt. Zur Öffnung des Reaktors läßt man über Nacht auf Raumtemperatur und dann auf -78 °C abkühlen. Dann läßt man auf Raumtemperatur kommen, überschüssiges Methylamin abdampfen, verdünnt mit Essigester, wäscht neutral, trocknet über Natriumsulfat, engt i.Vak. ein und chromatographiert an Kieselgel mit Dichlormethan / Methanol. Man erhält 14,8 g Methyl-[3-(4,4,5,5,5-pentafluorpentansulfonyl)-propyl]-amin als Kristalle vom Schmelzpunkt 55-57 °C.

**Patentansprüche**

1. 7α-(5-Methylaminopentyl)-estratriene der allgemeinen Formel I

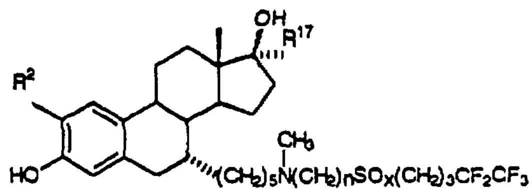

worin

$R^2$ für ein Wasserstoff- oder Fluoratom,
$R^{17}$ für ein Wasserstoffatom, eine Methyl- oder Ethinylgruppe,
n für 2, 3 oder 4, und
x für 0, 1 oder 2

stehen.

2. Estratriene nach Anspruch 1, worin $R^2$ ein Wasserstoffatom ist.

3. Estratriene nach Anspruch 1, worin $R^2$ ein Fluoratom ist.

4. Estratriene nach Anspruch 1, worin $R^{17}$ für ein Wasserstoffatom steht.

5. Estratriene nach Anspruch 1, worin $R^{17}$ für eine Methylgruppe steht.

6. Estratriene nach Anspruch 1, worin $R^{17}$ für eine Ethinylgruppe steht.

7. Estratriene nach Anspruch 1, worin n für 2 steht.

8. Estratriene nach Anspruch 1, worin n für 3 steht.

9. Estratriene nach Anspruch 1, worin n für 4 steht.

10. Estratriene nach Anspruch 1, worin x für 0 steht.

11. Estratriene nach Anspruch 1, worin x für 1 steht.

12. Estratriene nach Anspruch 1, worin x für 2 steht.

13. Estratriene nach Anspruch 1, nämlich

7α-{5-[N-Methyl-N-3-(4,4,5,5,5-pentafluorpentylthio)-propylamino]-pentyl}-estra-1,3,5(10)-trien-3,17β-diol
17α-Methyl-7α-{5-[N-methyl-N-3-(4,4,5,5,5-pentafluor-pentylthio)-propylamino]-pentyl}-estra-1,3,5(10)-trien-3,17β-diol
17α-Ethinyl-7α-{5-[N-methyl-N-3-(4,4,5,5,5-pentafluorpentylthio)-propylamino]-pentyl}-estra-1,3,5(10)-trien-3,17β-diol
7α-{5-[N-Methyl-N-2-(4,4,5,5,5-pentafluorpentansulfonyl)-ethylamino]-pentyl}-estra-1,3,5(10)-trien-3,17β-diol
7α-{5-[N-Methyl-N-3-(4,4,5,5,5-pentafluor-pentansulfonyl)-propylamino]-pentyl}-estra-1,3,5(10)-trien-3,17β-diol
2-Fluor-7α-{5-[N-Methyl-N-3-(4,4,5,5,5-pentafluorpentylthio)-propylamino]-pentyl}-estra-1,3,5(10)-trien-3,17β-diol
7α-{5-[N-Methyl-N-3-(4,4,5,5,5-pentafluorpentansulfinyl)-propylamino]-pentyl}-estra-1,3,5(10)-trien-3,17β-diol
2-Fluor-17α-methyl-7α-{5-[N-Methyl-N-3-(4,4,5,5,5-pentafluorpentylthio)-propylamino]-pentyl}-estra-1,3,5(10)-trien-3,17β-diol
2-Fluor-7α-{5-[N-Methyl-3-(4,4,5,5,5-pentafluorpentansulfinyl)-propylamino]-pentyl}-estra-1,3,5(10)-trien-3,17β-diol
2-Fluor-17α-methyl-7α-{5-[N-Methyl-N-3-(4,4,5,5,5-pentafluorpentansulfonyl)-propylamino]-pentyl}-estra-1,3,5(10)-trien-3,17β-diol
7α-{5-[N-Methyl-N-4-(4,4,5,5,5-pentafluorpentylthio)-butylamino]-pentyl}-estra-1,3,5(10)-trien-3,17β-diol
7α-{5-[N-Methyl-N-4-(4,4,5,5,5-pentafluorpentansulfinyl)-butyl am i no]-pentyl}-estra-1,3,5(10)-trien-3,17β-diol
17a-Methyl-7α-{5-[N-methyl-N-3-(4,4,5,5,5-pentafluorpentansulfinyl)-propylamino]-pentyl}-estra-1,3,5(10)-trien-3,17β-diol
17a-Methyl-7α-{5-[N-methyl-N-3-(4,4,5,5,5-pentafluorpentansulfonyl)-propylamino]-pentyl}-estra-1,3,5(10)-trien-3,17β-diol
17a-Ethinyl-7α-{5-[N-methyl-N-3-(4,4,5,5,5-pentafluorpentansulfonyl)-propylamino]-pentyl}-estra-1,3,5(10)-trien-3,17β-diol

**14.** Pharmazeutische Präparate enthaltend mindestens eine Verbindung der allgemeinen Formel I gemäß Anspruch 1 sowie einen pharmazeutisch verträglichen Träger.

**15.** Verwendung der Verbindungen der allgemeinen Formel 1 gemäß Anspruch 1 zur Herstellung von Arzneimitteln.

## Claims

**1.** 7α-(5-Methylaminopentyl)oestratrienes of the general formula I

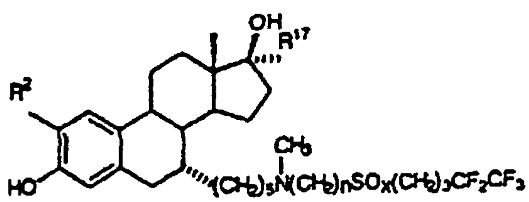

in which

$R^2$ is a hydrogen or fluorine atom,
$R^{17}$ is a hydrogen atom, or a methyl or ethynyl group,
n is 2, 3 or 4 and
x is 0, 1 or 2.

**2.** Oestratrienes according to Claim 1, in which $R^2$ is a hydrogen atom.

**3.** Oestratrienes according to Claim 1, in which $R^2$ is a fluorine atom.

**4.** Oestratrienes according to Claim 1, in which $R^{17}$ is a hydrogen atom.

**5.** Oestratrienes according to Claim 1, in which $R^{17}$ is a methyl group.

**6.** Oestratrienes according to Claim 1, in which $R^{17}$ is an ethynyl group.

**7.** Oestratrienes according to Claim 1, in which n is 2.

**8.** Oestratrienes according to Claim 1, in which n is 3.

**9.** Oestratrienes according to Claim 1, in which n is 4.

**10.** Oestratrienes according to Claim 1, in which x is 0.

**11.** Oestratrienes according to Claim 1, in which x is 1.

**12.** Oestratrienes according to Claim 1, in which x is 2.

**13.** Oestratrienes according to Claim 1, namely

7α-{5-[N-methyl-N-3-(4,4,5,5,5-pentafluoropentylthio)propylamino]pentyl}oestra-1,3,5(10)triene-3,17β-diol
17α-methyl-7α-{5-[N-methyl-N-3-(4,4,5,5,5-pentafluoropentylthio)propylamino]pentyl}oestra-1,3,5(10)triene-3,17β-diol
17α-ethynyl-7α-{5-[N-methyl-N-3-(4,4,5,5,5-pentafluoropentylthio)propylamino]-pentyl}oestra-1,3,5(10)triene-3,17β-diol
7α-{5-[N-methyl-N-2-(4,4,5,5,5-pentafluoropentanesulphonyl)ethylamino]pentyl}oestra-1,3,5(10)triene-3,17β-diol
7α-{5-[N-methyl-N-3-(4,4,5,5,5-pentafluoropentasulphonyl)propylamino]pentyl}oestra-1,3,5(10)triene-3,17β-diol
2-fluoro-7α-{5-[N-methyl-N-3-(4,4,5,5,5-pentafluoropentylthio)propylamino]pentyl}oestra-1,3,5(10)triene-3,17β-diol
7α-{5-[N-methyl-N-3-(4,4,5,5,5-pentafluoropentanesulphinyl)propylamino]pentyl}oestra-1,3,5(10)triene-3,17β-diol
2-fluoro-17α-methyl-7α-{5-[N-methyl-N-3-(4,4,5,5,5-pentafluoropentylthio)propylamino]pentyl}oestra-1,3,5(10)triene-3,17β-diol
2-fluoro-7α-{5-[N-methyl-3-(4,4,5,5,5-pentafluoropentanesulphinyl)propylamino]pentyl}oestra-1,3,5(10)triene-3,17β-diol
2-fluoro-17α-methyl-7α-{5-[N-methyl-N-3-(4,4,5,5,5-pentafluoropentanesulphonyl)propylamino]pentyl}oestra-1,3,5(10)triene-3,17β-diol
7α-{5-[N-methyl-N-4-(4,4,5,5,5-pentafluoropentylthio)butylamino]pentyl}oestra-1,3,5(10)triene-3,17β-diol
7α-{5-[N-methyl-N-4-(4,4,5,5,5-pentafluoropentanesulphonyl)butylamino]pentyl}oestra-1,3,5(10)triene-3,17β-diol
17α-methyl-7α-{5-[N-methyl-N-3-(4,4,5,5,5-pentafluoropentanesulphinyl)propylamino]pentyl}oestra-1,3,5(10)triene-3,17β-diol
17α-methyl-7α-{5-[N-methyl-N-3(4,4,5,5,5-pentafluoropentanesulphonyl)propylamino]pentyl}oestra-1,3,5(10)triene-3,17β-diol
17α-ethynyl-7α-{5-[N-methyl-N-3-(4,4,5,5,5-pentafluoropentanesulphonyl)propylamino]pentyl}oestra-1,3,5(10)triene-3,17β-diol

**14.** Pharmaceutical preparations comprising at least one compound of the general formula I according to Claim 1 and a pharmaceutically tolerable carrier.

**15.** Use of the compounds of the general formula I according to Claim 1 for the production of medicaments.

## Revendications

**1.** 7α-(5-méthylaminopentylestratriènes de formule générale I

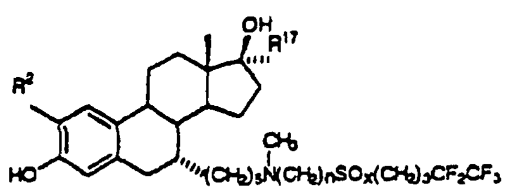

dans laquelle

$R^2$ représente un atome d'hydrogène ou de fluor,
$R^{17}$ représente un atome d'hydrogène, le groupe méthyle ou éthynyle,
n est 2, 3 ou 4, et
x est 0, 1 ou 2.

**2.** Estratriènes selon la revendication 1, dans lesquels $R^2$ est un atome d'hydrogène.

**3.** Estratriènes selon la revendication 1, dans lesquels $R^2$ est un atome de fluor.

**4.** Estratriènes selon la revendication 1, dans lesquels $R^{17}$ est un atome d'hydrogène.

**5.** Estratriènes selon la revendication 1, dans lesquels $R^{17}$ est le groupe méthyle.

**6.** Estratriènes selon la revendication 1, dans lesquels $R^{17}$ est le groupe éthynyle.

**7.** Estratriènes selon la revendication 1, dans lesquels n est 2.

**8.** Estratriènes selon la revendication 1, dans lesquels n est 3.

**9.** Estratriènes selon la revendication 1, dans lesquels n est 4.

**10.** Estratriènes selon la revendication 1, dans lesquels x est 0.

**11.** Estratriènes selon la revendication 1, dans lesquels x est 1.

**12.** Estratriènes selon la revendication 1, dans lesquels x est 2.

**13.** Estratriènes selon la revendication 1, à savoir

7α-{5-[N-méthyl-N-3-(4,4,5,5,5-pentafluoropentylthio)-propylamino]-pentyl}-estra-1,3,5(10)-triène-3,17β-diol
17α-méthyl-7α-{5-[N-méthyl-N-3-(4,4,5,5,5-pentafluoropentylthio)-propylamino]-pentyl}-estra-1,3,5(10)-triène-3,17β-diol
17α-éthynyl-7α-{5-[N-méthyl-N-3-(4,4,5,5,5-pentafluoropentylthio)-propylamino]-pentyl}-estra-1,3,5(10)-triène-3,17β-diol
7α-{5-[N-méthyl-N-2-(4,4,5,5,5-pentafluoropentanesulfonyl)-éthylamino]-pentyl}-estra-1,3,5(10)-triène-3,17β-diol
7α-{5-[N-méthyl-N-3-(4,4,5,5,5-pentafluoropentanesulfonyl)-propylamino]-pentyl}-estra-1,3,5(10)-triène-3,17β-diol
2-fluoro-7α-{5-[N-méthyl-N-3-(4,4,5,5,5-pentafluoropentylthio)-propylamino]-pentyl}-estra-1,3,5(10)-triène-3,17β-diol
7α-{5-[N-méthyl-N-3-(4,4,5,5,5-pentafluoropentanesulfinyl)-propylamino]-pentyl}-estra-1,3,5(10)-triène-3,17β-diol
2-fluoro-17α-méthyl-7α-{5-[N-méthyl-N-3-(4,4,5,5,5-pentafluoropentylthio)-propylamino]-pentyl}-estra-1,3,5(10)-triène-3,17β-diol
2-fluoro-7α-{5-[N-méthyl-N-3-(4,4,5,5,5-pentafluoropentanesulfinyl)-propylamino]-pentyl}-estra-1,3,5(10)-triène-3,17β-diol
2-fluoro-17α-méthyl-7α-{5-[N-méthyl-N-3-(4,4,5,5,5-pentafluoropentanesulfonyl)-propylamino]-pentyl}-estra-1,3,5(10)-triène-3,17β-diol
7α-{5-[N-méthyl-N-4-(4,4,5,5,5-pentafluoropentylthio)-butylamino]-pentyl}-estra-1,3,5(10)-triène-3,17β-diol
7α-{5-[N-méthyl-N-4-(4,4,5,5,5-pentafluoropentanesulfinyl)-butylamino]-pentyl}-estra-1,3,5(10)-triène-3,17β-diol
17α-méthyl-7α-{5-[N-méthyl-N-3-(4,4,5,5,5-pentafluoropentanesulfinyl)-propylamino] -pentyl}-estra-1,3,5(10)-triène-3,17β-diol
17α-méthyl-7α-{5-[N-méthyl-N-3-(4,4,5,5,5-pentafluoropentanesulfonyl)-propylamino]-pentyl}-estra-1,3,5(10)-triène-3,17β-diol
17α-éthynyl-7α-{5-[N-méthyl-N-3-(4,4,5,5,5-pentafluoropentanesulfonyl)-propylamino]-pentyl}-estra-1,3,5(10)-triène-3,17β-diol.

**14.** Préparations pharmaceutiques contenant au moins un composé de formule générale I selon la revendication 1, ainsi qu'un véhicule pharmaceutiquement acceptable.

**15.** Utilisation des composés de formule générale I selon la revendication 1, pour la fabrication de médicaments.

## Abb. 1

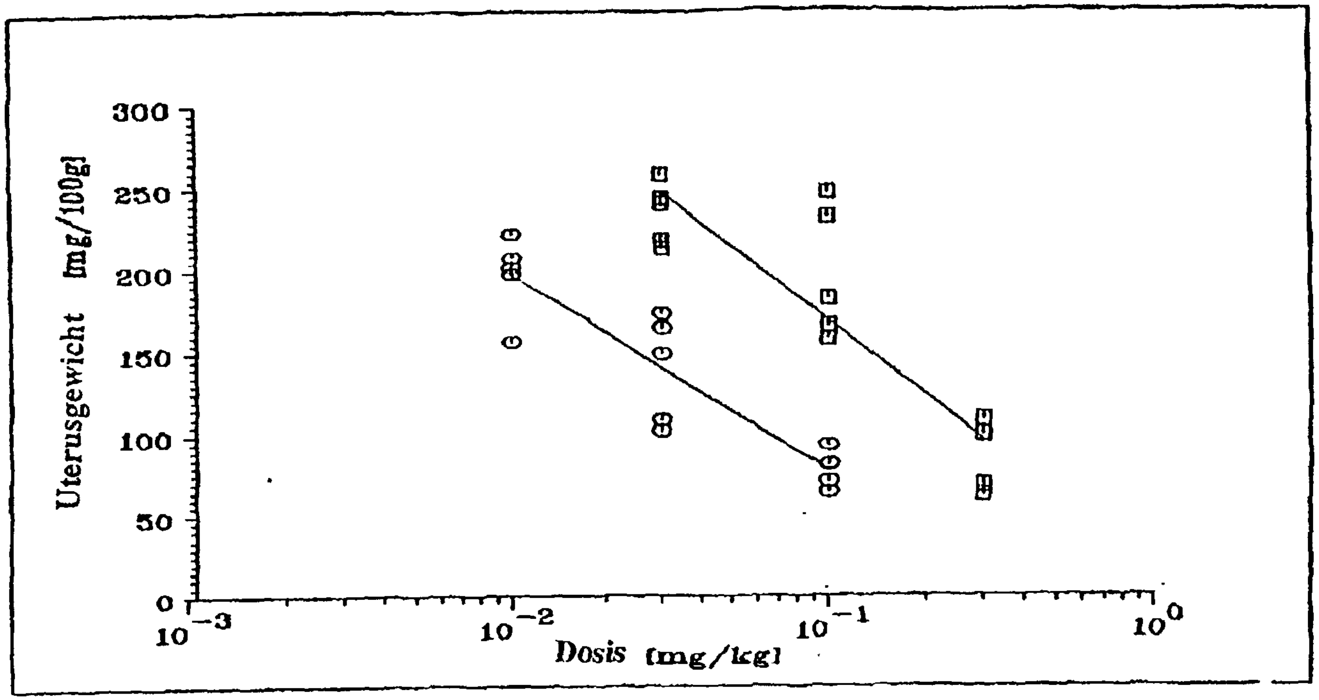


**Antiuterotrope Wirkung bei der infantilen Ratte s.c.**

□ 7α-[9-(4,4,5,5,5-Pentafluorpentylsulfinyl)-n-nonyl]-estra-1,3,5(10)-trien-3,17β-diol

○ 7α-{5-[N-Methyl-N-3-(4,4,5,5,5-pentafluorpentylthio)-propylamino]-pentyl}-estra-1,3,5(10)-trien-3,17β-diol

Relative Wirksamkeiten 5.4

**Abb. 2**

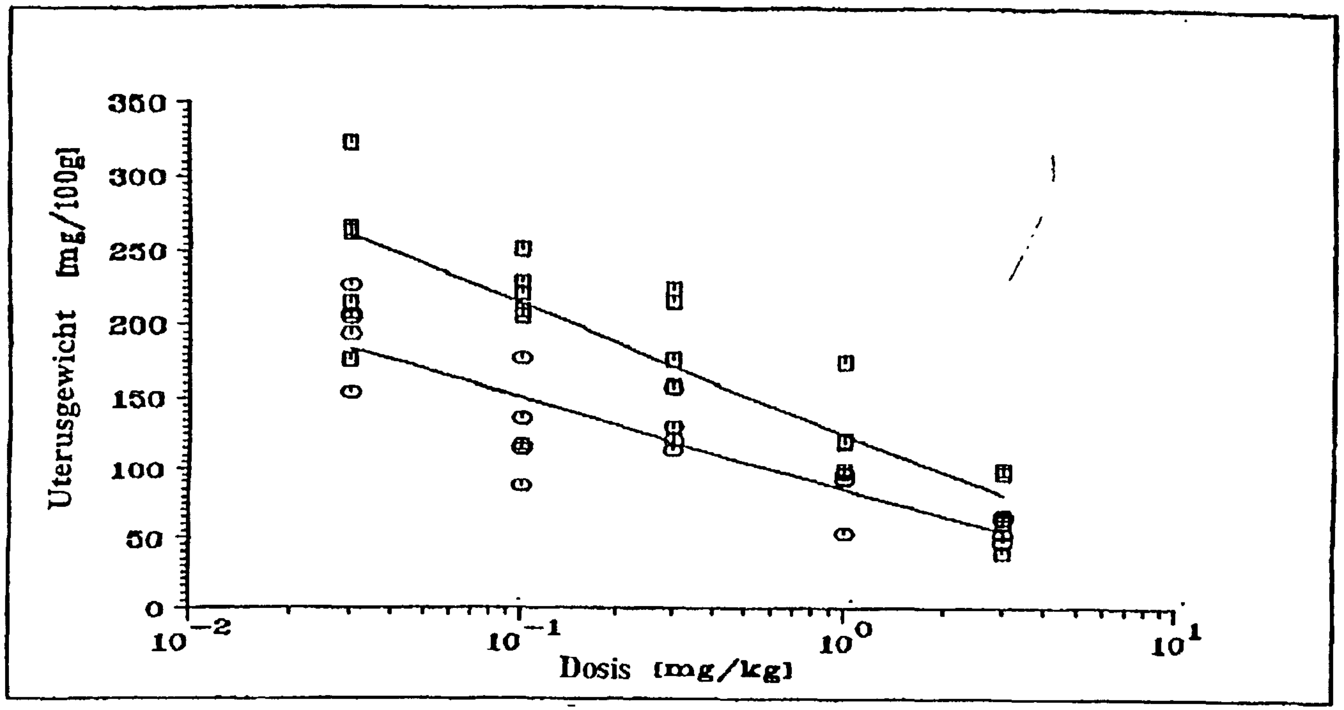


Antiuterotrope Wirkung bei der infantilen Ratte p.o.

⊟ 7α-[9-(4,4,5,5,5-Pentafluorpentylsulfinyl)-n-nonyl]-estra-1,3,5(10)-trien-3,17β-diol

⊖ 7α-{5-[N-Methyl-N-3-(4,4,5,5,5-pentafluorpentylthio)-propylamino]-pentyl}-estra-1,3,5(10)-trien-3,17β-diol

Relative Wirksamkeiten 4,7

Abb. 3

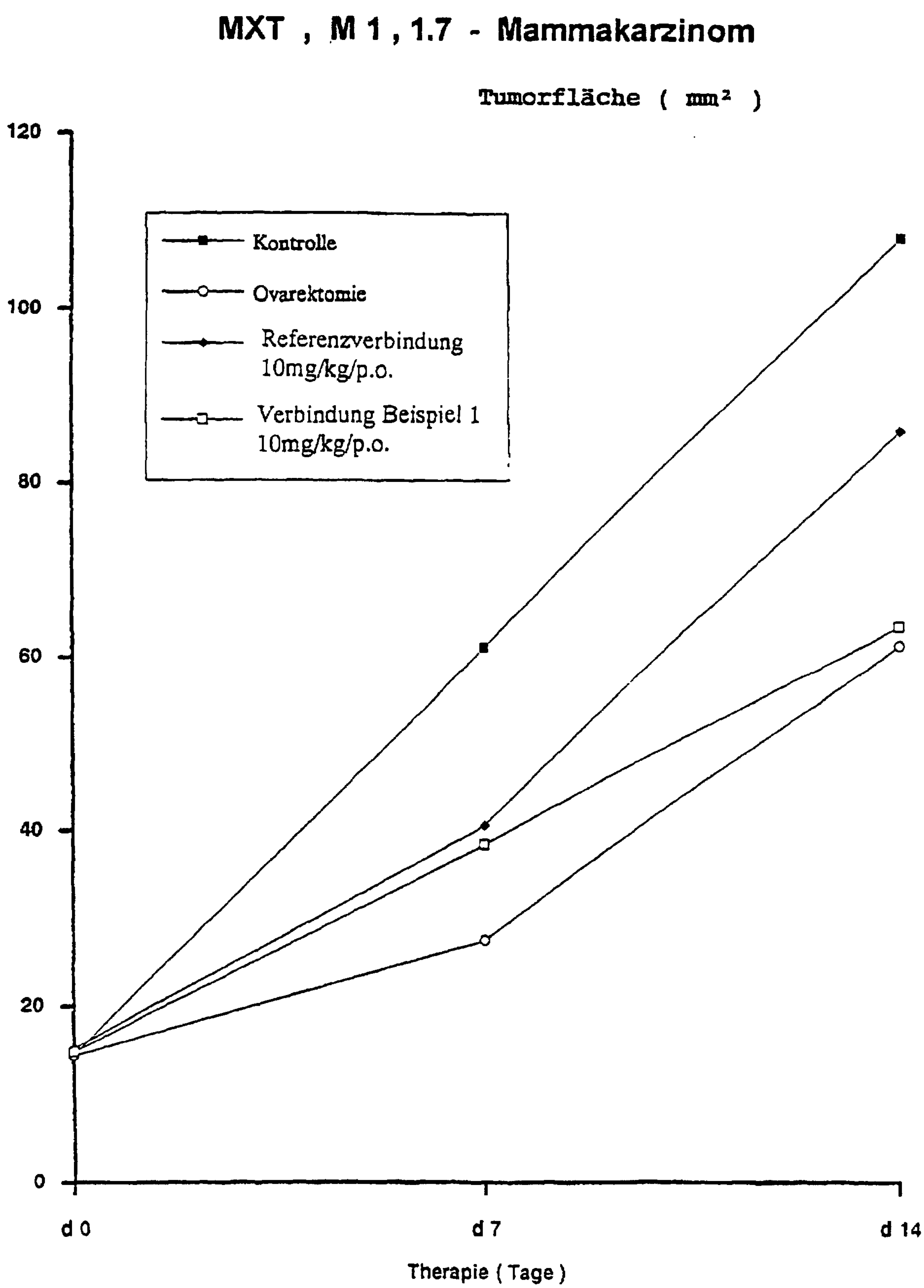
MXT , M 1 , 1.7 - Mammakarzinom
Tumorfläche ( mm² )
120
100
80
60
40
20
0
Kontrolle
Ovarektomie
Referenzverbindung 10mg/kg/p.o.
Verbindung Beispiel 1 10mg/kg/p.o.
d 0
d 7
d 14
Therapie ( Tage )

DMBA - induzierte Mamma Tumore p.o. Abb. 4

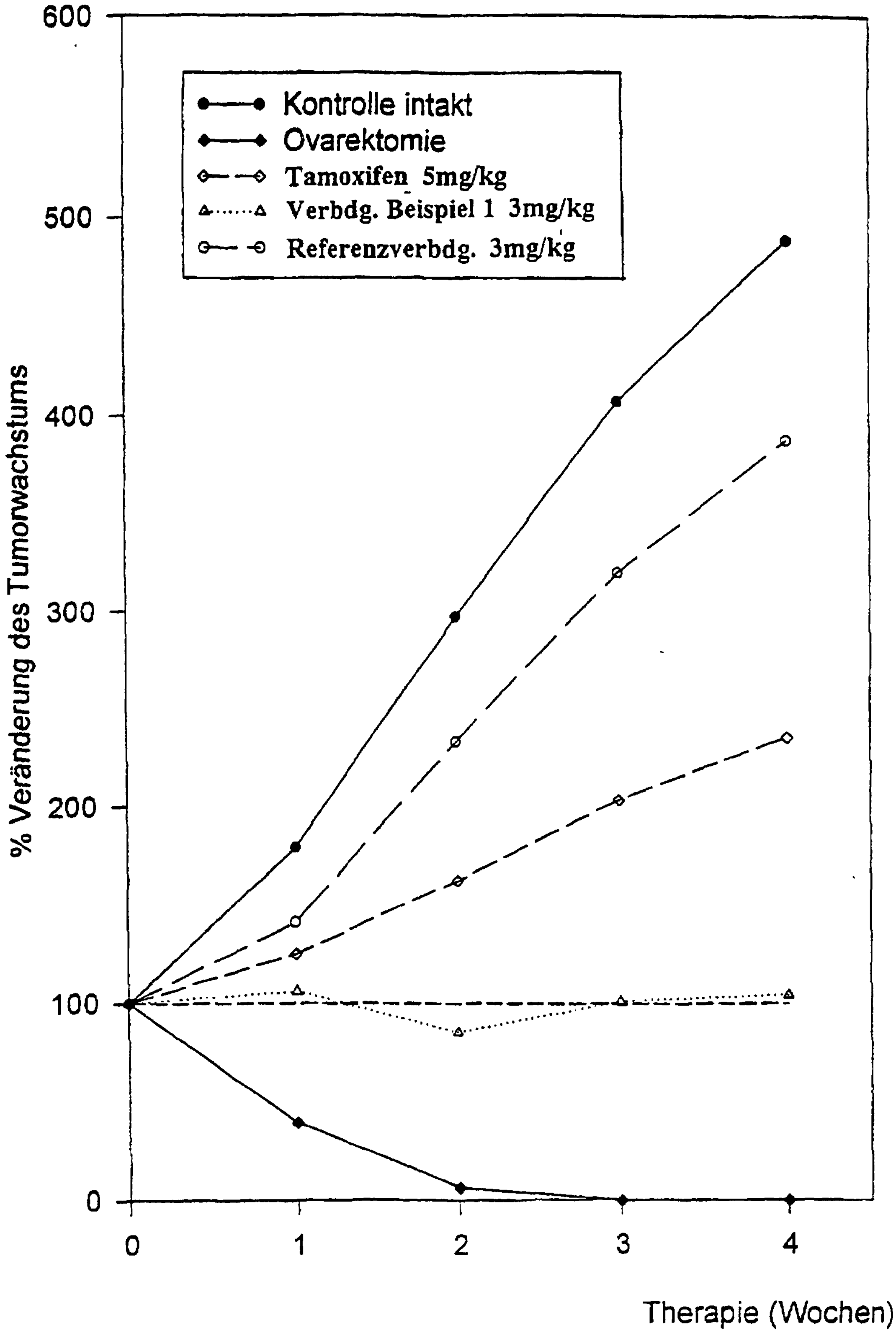

NMU - induzierte Mamma Tumore p.o. Abb.5